**Europäisches Patentamt**

**European Patent Office**

**Office européen des brevets**

(19)

(11) Publication number: **0 195 087**
**A1**

## (12) EUROPEAN PATENT APPLICATION
published in accordance with Art. 158(3) EPC

(21) Application number: **85904851.4**

(22) Date of filing: **18.09.85**

Data of the international application taken as a basis:

(86) International application number:
**PCT/JP85/00518**

(87) International publication number:
**WO86/01828 (27.03.86 86/07)**

(51) Int. Cl.⁴: **C 12 N 15/00**
C 12 N 1/20, C 12 P 21/00
//A61K39/13, (C12N1/20,
C12R1:19), (C12P21/00,
C12R1:19)

(30) Priority: **18.09.84 JP 193869/84**
**20.03.85 JP 54236/85**

(43) Date of publication of application:
**24.09.86 Bulletin 86/39**

(84) Designated Contracting States:
**DE FR**

(71) Applicant: **CENTRAL GLASS COMPANY, LIMITED**
**5253, Oaza Okiube Ube-shi**
**Yamaguchi 755(JP)**

(71) Applicant: **SAGAMI CHEMICAL RESEARCH CENTER**
**4-5, Marunouchi 1-chome**
**Chiyoda-ku, Tokyo, 100(JP)**

(71) Applicant: **HODOGAYA CHEMICAL CO., LTD.**
**4-2, Toranomon 1 Chome Minato-ku**
**Tokyo(JP)**

(71) Applicant: **NISSAN CHEMICAL INDUSTRIES LTD.**
**3-7-1, Kanda Nishiki-cho**
**Chiyoda-ku Tokyo(JP)**

(71) Applicant: **TOYO SODA MANUFACTURING CO., LTD.**
**No. 4560, Oaza-tonda Shin-nanyo-shi**
**Yamaguchi-ken(JP)**

(71) Applicant: **NIPPON SODA CO., LTD.**
**2-1, Ohte-machi 2-chome**
**Chiyoda-ku, Tokyo, 100(JP)**

(72) Inventor: **NUMAO, Naganori**
**9-2, Minamidai 1-chome**
**Sagamihara-shi Kanagawa 228(JP)**

(72) Inventor: **TAKAHARA, Yoshiyuki**
**16, Sakaecho 3-chome**
**Sagamihara-shi Kanagawa 228(JP)**

(72) Inventor: **KATO, Seishi**
**9-1, Minamidai 1-chome**
**Sagamihara-shi Kanagawa 228(JP)**

(74) Representative: **Patentanwälte Grünecker, Kinkeldey,**
**Stockmair & Partner**
**Maximilianstrasse 58**
**D-8000 München 22(DE)**

(54) **CYCLIC DOUBLE-STRANDED DNA, PROCESS FOR ITS PREPARATION, MICROORGANISM CONTAINING SAID CYCLIC DOUBLE-STRANDED DNA, AND PROCESS FOR PREPARING PROTEIN CONTAINING ANTIGEN DETERMINANT SITE OF POLIOVIRUS CAPSIDE PROTEIN VP1 USING THE SAME.**

(57) A process for preparing a protein having an antigen determinant site of capside protein VPI utilizing cyclic double-stranded DNA containing high expression promotor/operator, SD sequence under its control, and a DNA portion which codes an antigen determinant site of capside protein VPI of attenuated poliovirus strain. This invention enables to produce the protein in a large amount as a blended or non-blended protein.

./...

Fig. 1

0195087

## Specification

Cyclic double-stranded DNA and process for producing the same, microorganism containing said cyclic double-stranded DNA, and process for preparing protein having antigenic determinant of poliovirus capsid protein VP1 by use of them

[Technical field]

This invention relates to a production technique, utilizing the recombinant gene technique, of a protein having the antigenic determinant of the capsid protein VP1 of poliovirus which is a pathogen of acute anterior polyomyelitis.

[Background art]

At present, immunization against acute anterior polyomyelitis is effected by parenteral administration of poliovirus which has been treated with formalin or other chemical reagent to lose its infectiousness while permitting its antigenicity to remain, namely an inactivated vaccine, or by oral administration of attenuated poliovirus itself, namely live vaccine.

Of these, the inactivated vaccine requires a large amount of virus for immunization and therefore high in cost, and also involves the drawback that immunization cannot be maintained over the whole life.

On the other hand, live vaccine has the probability that a highly pathogenic strain may arise through mutation which may occur on the virus gene after repeated proliferation. For this reason, there are involved the problems of danger of outbreak of disease by inoculation of live vaccine and exhaustion of seed virus, particularly Sabin type 3. Also, it has the problems such as danger of administration to immuno-deficient people, contaminant of monkey tumor virus by use of monkey cells, difficult availability of monkey under emergent circumstances, exhaustion and high cost of monkey source in the future.

It has also been attempted to produce an immunizing protein in E. coli cells in place of live vaccine. That is, van der Werf, S., et al [Gene, 23, 85 (1983)] and Marr Jirow (Japanese Unexamined Patent Publication No. 173084/1984) produced the capsid protein VP1 in the form of a fused protein by expression of cDNA coading for the capsid protein VP1 of type 1 strongly poisonous strain Mahoney type in E. coli. It is described there that corresponding DNA sequences of attenuated strains such as Sabin type 1, 2 and 3 may also be used, but neither specific means nor possibility of practice is shown. On the other hand, Kuge, S., et al [Journal of Biochemistry, 96, 305 (1984)] produced the capsid protein VP1 as the fused protein by expression of the cDNA coding for the capsid protein VP1 of Sabin type 1 incorporated in a plasmid containing an ampicillin resistant gene by use of trp promoter. However, in both of these methods, the amount of VP1 expressed is very minute, and it would be desirable to have an emergence of a method capable of producing a large amount of the capsid protein VP1.

The present inventors studied intensively in order to cancel the problems of the prior art as mentioned above, and consequently successfully produced a protein having an antigenic determinant of the specific protein of poliovirus in a large amount to accomplish the present invention.

In the present specification, non-fused means no fusion with a polypeptide other than the proteins derived from polyovirus, and the VP3 protein of poliovirus (encoded. before VP1) may be attached, or the VP1 protein containing a part from its intermediate portion may be also included.

The present invention is intended to provide a plasmid and a microorganism capable of producing a protein having the antigenic determinant of the capsid protein VP1 of the attenuated strain of poliovirus as the fused protein or in the non-fused form, and a process for producing said protein by use thereof.

[Disclosure of the invention]

A first cyclic double-stranded DNA of the present invention comprises a high expression promoter-operator, a DNA region coding for the amino group end portion of the metapyrocatechase gene containing the SD sequence of the metapyrocatechase gene positioned in the downstream region under the control thereof and a DNA portion subsequent thereto coding for a peptite corresponding to at least the amino acid number 92 to 105 of the capsid protein VP1 of the poliovirus attenuated strain Sabin type 1, Sabin type 2 or Sabin type 3.

A second cyclic double-stranded DNA of the present invention comprises a high expression promoter-operator, a SD sequence positioned in the downstream region under the control thereof, a translation initiation codon, a DNA portion connected thereto in series so as to coincide in reading frame coding for a peptide corresponding to at least the the amino acid number 92 to 105 of the capsid protein VP1 of the poliovirus attenuated strain Sabin type 1, Sabin type 2 or Sabin type 3 and a translation termination codon subsequent thereto.

Examples of the above high expression promoter-operator include phage λ initial gene groups ($P_L$) promoter-operator, <u>lac</u> UV5 promoter-operator, <u>trp</u> promoter-operator and <u>tac</u> promoter-operator.

$P_L$ promoter-operator is contained in, for example, the <u>Hind</u> III-<u>Bam</u> HI fragment derived from phage λ. Such a fragment may be excised directly from phage λ, but it may also be excised from another appropriate plasmid containing the fragment. Examples of such a plasmid include pKC30 plasmid [Nature, <u>292</u>, 128 (1981)] and pPL-10 plasmid (commercial products).

An example of the DNA sequence containing the $P_L$ promoter-operator is represented by the following formula (I):

```
                                                           50
5'··· GATCTCTCACCTACCAAACAATGCCCCCCTGCAAAAAATAAATTCATATA
     XXXXXXXXXXXXXXXXXXXXXXXXXXXXXXXXXXXXXXXXXXXXXXXXXX

                                                          100
     AAAAACATACAGATAACCATCTGCGGTGATAAATTATCTCTGGCGGTGTT
     XXXXXXXXXXXXXXXXXXXXXXXXXXXXXXXXXXXXXXXXXXXXXXXXXX

                                                          150
     GACATAAATACCACTGGCGGTGATACTGAGCACATCAGCAGGACGCACTG
     XXXXXXXXXXXXXXXXXXXXXXXXXXXXXXXXXXXXXXXXXXXXXXXXXX

                                                          200
     ACCACCATGAAGGTGACGCTCTTAAAAATTAAGCCCTGAAGAAGGGCAGC   (I)
     XXXXXXXXXXXXXXXXXXXXXXXXXXXXXXXXXXXXXXXXXXXXXXXXXX
```

250

ATTCAAAGCAGAAGGCTTTGGGGTGTGTGATACGAAACGAAGCATTGGCC

XXXXXXXXXXXXXXXXXXXXXXXXXXXXXXXXXXXXXXXXXXXXXXXXXXX

300

GTAAGTGCGATTCCGGATTAGCTGCCAATGTGCCAATCGCGGGGGGTTTT

XXXXXXXXXXXXXXXXXXXXXXXXXXXXXXXXXXXXXXXXXXXXXXXXXXX

350

CGTTCAGGACTACAACTGCCACACACCACCAAAGCTAACTGACAGGAGAA

XXXXXXXXXXXXXXXXXXXXXXXXXXXXXXXXXXXXXXXXXXXXXXXXXXX

400

TCCAGATGGATGCACAAACACGCCGCCGCGAACGTCGCGCAGAGAAACAG

XXXXXXXXXXXXXXXXXXXXXXXXXXXXXXXXXXXXXXXXXXXXXXXXXXX

450

GCTCAATGGAAAGCAGCAAATCCCCTGTTGGTTGGGGTAAGCGCAAAACC

XXXXXXXXXXXXXXXXXXXXXXXXXXXXXXXXXXXXXXXXXXXXXXXXXXX


AGTT······3'

XXXX
    (wherein A represents a deoxyadenylic acid group, G a
    deoxyguanylic acid group, C a deoxycitidylic acid
    group, T a thymidilic acid group, X represents T when
    the corresponding base in the other single-stranded
    DNA is A, or A when it is T, or G when it is C, or C
    when it is G).

Said DNA sequence contains a DNA sequence corresponding to the <u>Bgl</u> II - <u>Hpa</u>I fragment of the <u>Hind</u> III - <u>Bam</u> HI fragment containing the $P_L$ promoter-operator.

The DNA sequence coding for the amino group terminus (hereinafter called "N-terminus") of the metapyrocatechase gene containing the SD sequence of the metapyrocatechase gene in the first cyclic double-stranded DNA is contained in, for example, the TOL plasmid of <u>Pseudomonas putida</u> mt - 2 strain (ATCC 23973). Such a DNA fragment may be excised as the <u>SsT</u> II directly from the TOL plasmid or it may also be excised from another appropriate plasmid containing the fragment. Examples of such a plasmid include pTS 115 plasmid [Inoue, S. et al; Journal of Bacteriology (J. Bacteriol.), <u>145</u>, 1137 (1981)] and pYT3 plasmid [Ebina, Y., et al; Journal of Bacteriology (J. Bacteriol.), <u>156</u>, 487 (1983)].

An example of the DNA sequence coding fo the N-terminus of the metapyrocatechase gene containing said SD sequence is the DNA sequence of which single strand is shown by the following formula (II):

5'··· AATTCCGGTCACGTGCCGGCCAAGGCCGCCGCCGAAGGCTGGCCCTGGCC

TGTCAAGTGTTTCCGCAAACCGACTTGACCATCGAGTACTTCGCCACGTT        ( II )

GGCGGAAACAAACCTGACAACATGAACTATGAAGAGGTGACGTCATGAAC

AAAGGTGTAATGCGACCGGGCCATGTGCAG···3'

(wherein A, G, C and T have the same meanings as defined above).

The SD sequence in the second cyclic double-stranded DNA in the present invention may include the SD sequence of the metapyrocatechase gene, the SD sequence of λcII, the SD sequence of λcro, the SD sequence of the lacZ gene, the SD sequence of the trpL, and the SD sequence of the lpp gene, of which the SD sequence of the metapyrocatechase gene is preferred.

The DNA sequence containing the SD sequence of the meta-catechase gene and the translation initiation codon ATG can be obtained by preparing this portion according to, for example, chemical synthesis, and recombining said DNA fragment with a suitable vector for expression having a high expression promoter-operator. Alternatively, it may be prepared according to a genetic engineering method from a high expression promoter-operator and a vector for expression containing the SD sequence of the metapyrocatechase gene. Examples of such a vector for expression include the pHT 3 plasmids (deposited at Institute of Fermentation Research, Agency of Industrial Science & Technology, Ministry of International Trade & Industry (hereinafter abbreviated as "FERM") as Escherchia coli HB 101/pHT3 FERM P-7776, Escherchia coli C 600/pHT3 FERM P-7777, and Escherchia coli W 3110/pHT3 FERM P-7778, and pHT 311 plasmid (deposited at FERM as Escherchia coli HB 101/pHT3 FERM P-7994).

Further, an example of the vector having the SD sequence of the metapyrocatechase gene and the translation initiation codon, and also having a cleavable restriction endonuclease cleaving site thereafter is the pYTU3 plasmid (deposited at FERM as Escherchia coli HB 101/pYTU3 FERM P-7992).

The DNA sequence in the SD sequence and the initiation codon region of the plasmid pYTU3 is represented by the following formula (III):

```
TGAAGAGGTGCATCG ATG TCG A
                                        (III)
ACTTCTCCACGTAGC TAC AGC T
SD sequence   Cla I    Sal I
```

(wherein A, G, C and T have the same meanings as
defined above).

In the present invention, the portion coding for a
peptide corresponding to at least the amino acid number
92 to 105 of the capsid protein VP1 of the poliovirus
attenuated strain Sabin type 1, Sabin type 2 or Sabin
type 3 may be any DNA sequence, provided that it can code
for the peptide represented by the following formula:

Val Asp Asn Ser Ala Ser Thr Lys Asn Lys Asp Lys Leu Phe;

Val Asp Asn Asp Ala Pro Thr Lys Arg Ala Ser Arg Leu Phe; or Val Asp Asn Glu Gln Pro Thr Thr Arg Ala Gln Lys Leu Phe;

Of these DNA sequences, those related to Sabin type 1 are
contained in the Taq I fragment, the Taq I - Kpn I
fragment and the Bam HI - Hinc II fragment derived from
the PBB2 plasmid [Nomoto, Akio, et al; (Proceeding
National Academic Science U.S.A. (Proc. Natl. Acad. Sci.
USA.), 79, 5793 (1982)].

The DNA sequence of said Taq I fragment is represented by
the following formula (IV):

```
                                              50
       .        .       .         .        .
5'···CGACACCCAGAGAGATGGACATCCTTGGTTTTGTGTCAGCGTGTAATGAC

     XXXXXXXXXXXXXXXXXXXXXXXXXXXXXXXXXXXXXXXXXXXXXXXXXXX
```

100

TTCAGCGTGCGCTTGATGCGAGATACCACACATATAGAGCAAAAAGCGCT

XXXXXXXXXXXXXXXXXXXXXXXXXXXXXXXXXXXXXXXXXXXXXXXXXXX

150

AGCACAGGGGGTTAGGTCAGATGCTTGAAAGCATGATTGACAACACAGTCC

XXXXXXXXXXXXXXXXXXXXXXXXXXXXXXXXXXXXXXXXXXXXXXXXXXX

200

GTGAAACGGTGGGGGGCGGCAACGTCTAGAGACGCTCTCCCAAACACTGAA

XXXXXXXXXXXXXXXXXXXXXXXXXXXXXXXXXXXXXXXXXXXXXXXXXXX

250

GCCAGTGGACCAGCACACTCCAAGGAAATTCCGGCACTCACCGCAGTGGA    ( IV )

XXXXXXXXXXXXXXXXXXXXXXXXXXXXXXXXXXXXXXXXXXXXXXXXXXX

300

AACTGGGGGCCACAAATCCACTAGTCCCTTCTGATACAGTGCAAACCAGAC

XXXXXXXXXXXXXXXXXXXXXXXXXXXXXXXXXXXXXXXXXXXXXXXXXXX

350

ATGTTGTACAACATAGGTCAAGGTCAGAGTCTAGCATAGAGTCTTTCTTC

XXXXXXXXXXXXXXXXXXXXXXXXXXXXXXXXXXXXXXXXXXXXXXXXXXX

400

GCGCGGGGTGCATGCGTGGCCATTATAACCGTGGATAACTCAGCTTCCAC

XXXXXXXXXXXXXXXXXXXXXXXXXXXXXXXXXXXXXXXXXXXXXXXXXXX

450

CAAGAATAAGGATAAGCTATTTACAGTGTGGAAGATCACTTATAAAGATA

XXXXXXXXXXXXXXXXXXXXXXXXXXXXXXXXXXXXXXXXXXXXXXXXXXX

500

CTGTCCAGTTACGGAGGAAATTGGAGTTCTTCACCTATTCTAGATTTGAT

XXXXXXXXXXXXXXXXXXXXXXXXXXXXXXXXXXXXXXXXXXXXXXXXXXX

550

ATGGAATTTACCTTTGTGGTTACTGCAAATTTCACTGAGACTAAGAATGG

XXXXXXXXXXXXXXXXXXXXXXXXXXXXXXXXXXXXXXXXXXXXXXXXXXX

600

GCATGCCTTAAATCAAGTGTACCAAATTATGTACGTACCACCAGGCGCTC

XXXXXXXXXXXXXXXXXXXXXXXXXXXXXXXXXXXXXXXXXXXXXXXXXXX

650

CAGTGCGAGAAATGGGACGACTACACATGGCAAACCTCATCAAATCCATC

XXXXXXXXXXXXXXXXXXXXXXXXXXXXXXXXXXXXXXXXXXXXXXXXXXX

700

AATCTTTTACACCTACGGAACAGCTCCAGCCCGGATCTCGGTACCGTATG

XXXXXXXXXXXXXXXXXXXXXXXXXXXXXXXXXXXXXXXXXXXXXXXXXXX

TTGGTATTT  ...3'

XXXXXXXXXGC

(wherein A, G, C, T and X have the same meanings as defined above);

the DNA sequence of said Taq I - Kpn I fragment is represented by the following formula (V):

50

5'...CGACACCCAGAGAGATGGACATCCTTGGTTTTGTGTCAGCGTGTAATGAC

XXXXXXXXXXXXXXXXXXXXXXXXXXXXXXXXXXXXXXXXXXXXXXXXXX

100

TTCAGCGTGCGCTTGATGCGAGATACCACACATATAGAGCAAAAAGCGCT

XXXXXXXXXXXXXXXXXXXXXXXXXXXXXXXXXXXXXXXXXXXXXXXXXXX

150

AGCACAGGGGTTAGGTCAGATGCTTGAAAGCATGATTGACAACACAGTCC

XXXXXXXXXXXXXXXXXXXXXXXXXXXXXXXXXXXXXXXXXXXXXXXXXXX

200

GTGAAACGGTGGGGGCGGCAACGTCTAGAGACGCTCTCCCAAACACTGAA

XXXXXXXXXXXXXXXXXXXXXXXXXXXXXXXXXXXXXXXXXXXXXXXXXXX

```
                                                    250
GCCAGTGGACCAGCACACTCCAAGGAAATTCCGGCACTCACCGCAGTGGA
XXXXXXXXXXXXXXXXXXXXXXXXXXXXXXXXXXXXXXXXXXXXXXXXXXX

                                                    300
AACTGGGGGCACAAATCCAGTAGTCCCTTCTGATACAGTGCAAACCAGAC
XXXXXXXXXXXXXXXXXXXXXXXXXXXXXXXXXXXXXXXXXXXXXXXXXXX

                                                    350
ATGTTGTACAACATAGGTCAAGGTCAGAGTCTAGCATAGAGTCTTTCTTC   (V)
XXXXXXXXXXXXXXXXXXXXXXXXXXXXXXXXXXXXXXXXXXXXXXXXXXX

                                                    400
GCGCGGGGTGCATGCGTGGCCATTATAACCGTGGATAACTCAGCTTCCAC
XXXXXXXXXXXXXXXXXXXXXXXXXXXXXXXXXXXXXXXXXXXXXXXXXXX

                                                    450
CAAGAATAAGGATAAGCTATTTACAGTGTGGAAGATCACTTATAAAGATA
XXXXXXXXXXXXXXXXXXXXXXXXXXXXXXXXXXXXXXXXXXXXXXXXXXX

                                                    500
CTGTCCAGTTACGGAGGAAATTGGAGTTCTTCACCTATTCTAGATTTGAT
XXXXXXXXXXXXXXXXXXXXXXXXXXXXXXXXXXXXXXXXXXXXXXXXXXXX
```

- 13 -

0195087

550

ATGGAATTTACCTTTGTGGTTACTGCAAATTTCACTGAGACTAACAATGG

XXXXXXXXXXXXXXXXXXXXXXXXXXXXXXXXXXXXXXXXXXXXXXXXXXX

600

GCATGCCTTAAATCAAGTGTACCAAATTATGTACGTACCACCAGGCGCTC

XXXXXXXXXXXXXXXXXXXXXXXXXXXXXXXXXXXXXXXXXXXXXXXXXXX

650

CAGTGCCCGAGAAATGGGACGACTACACATGGCAAACCTCATCAAATCCA

XXXXXXXXXXXXXXXXXXXXXXXXXXXXXXXXXXXXXXXXXXXXXXXXXXX

700

TCAATCTTTTACACCTACGGAACAGCTCCAGCCCGGATCTCGGTACCGTA

XXXXXXXXXXXXXXXXXXXXXXXXXXXXXXXXXXXXXXXXXXXXXXXXXXX

750

TGTTGGTATTTCGAACGCCTATTCACACTTTTACGACGGTTTTTCCAAAG

XXXXXXXXXXXXXXXXXXXXXXXXXXXXXXXXXXXXXXXXXXXXXXXXXXX

800

TACCACTGAAGGACCAGTCGGCAGCACTAGGTGACTCCCTCTATGGTGCA

XXXXXXXXXXXXXXXXXXXXXXXXXXXXXXXXXXXXXXXXXXXXXXXXXXX

0195087

850

GCATCTCTAAATGACTTCGGTATTTTGGCTGTTAGAGTAGTCAATGATCA
XXXXXXXXXXXXXXXXXXXXXXXXXXXXXXXXXXXXXXXXXXXXXXXXXXX

900

CAACCCGACCAAGGTCACCTCCAAAATCAGAGTGTATCTAAAACCCAAAC
XXXXXXXXXXXXXXXXXXXXXXXXXXXXXXXXXXXXXXXXXXXXXXXXXXX

950

ACATCAGAGTCTGGTGCCCGCGTCCACCGAGGGCAGTGGCGTACTACGGC
XXXXXXXXXXXXXXXXXXXXXXXXXXXXXXXXXXXXXXXXXXXXXXXXXXX

1000

CCTGGAGTGGATTACAAGGATGGTACGCTTACACCCCTCTCCACCAAGGA
XXXXXXXXXXXXXXXXXXXXXXXXXXXXXXXXXXXXXXXXXXXXXXXXXXX

1050

TCTGACCACATATGGATTCGGACACCAAAACAAAGCGGTGTACACTGCAG
XXXXXXXXXXXXXXXXXXXXXXXXXXXXXXXXXXXXXXXXXXXXXXXXXXX

1100

GTTACAAAATTTGCAACTACCATTTGGCCACTCAGGAAGATTTGCAAAAC
XXXXXXXXXXXXXXXXXXXXXXXXXXXXXXXXXXXXXXXXXXXXXXXXXXX

GCAGTGAACGTCATGTGGAATAGAGACCTCTTAGTCACAGAATCAAGAGC

XXXXXXXXXXXXXXXXXXXXXXXXXXXXXXXXXXXXXXXXXXXXXXXXXXXX

CCAGGGCACCGATTCAATCGCAAGGTGCAATTGCAACGCAGGGGTGTACT

XXXXXXXXXXXXXXXXXXXXXXXXXXXXXXXXXXXXXXXXXXXXXXXXXXXX

ACTGCGAGTCTAGAAGGAAATACTACCCAGTATCCTTCGTTGGCCCAACG

XXXXXXXXXXXXXXXXXXXXXXXXXXXXXXXXXXXXXXXXXXXXXXXXXXXX

TTCCAGTACATGGAGGCTAATAACTATTACCCAGCTAGGTAC···3'

XXXXXXXXXXXXXXXXXXXXXXXXXXXXXXXXXXXXXXXX

(wherein A, G, C, T and X have the same meanings as
defined above);
the DNA sequence of said Bam HI - Hinc II fragment is
represented by the following formula (VI):

5'···GATCCATGATGGCAACTGGCAAACTGTTGGTGTCATACGCGCCTCCTGGA

XXXXXXXXXXXXXXXXXXXXXXXXXXXXXXXXXXXXXXXXXXXXXXXXXXXX

```
                                                        100
     .         .         .         .         .
GCCGACCCACCAAAGAAGCGTAAGGAGGCGATGTTGGGAACACATGTGAT
XXXXXXXXXXXXXXXXXXXXXXXXXXXXXXXXXXXXXXXXXXXXXXXXXXX

                                                        150
     .         .         .         .         .
CTGGGACATAGGACTGCAGTCCTCATGTACTATGGTAGTGCCATGGATTA
XXXXXXXXXXXXXXXXXXXXXXXXXXXXXXXXXXXXXXXXXXXXXXXXXXX

                                                        200
     .         .         .         .         .
GCAACACCACGTATCGGCAAACCATAGATGATAGTTTCACCGAAGGCGCA
XXXXXXXXXXXXXXXXXXXXXXXXXXXXXXXXXXXXXXXXXXXXXXXXXXX

                                                        250
     .         .         .         .         .
TACATCAGCGTCTTCTACCAAACCAGAATAGTCGTCCCTCTTTCGACACC
XXXXXXXXXXXXXXXXXXXXXXXXXXXXXXXXXXXXXXXXXXXXXXXXXXX

                                                        300
     .         .         .         .         .
CAGAGAGATGGACATCCTTGGTTTTGTGTCAGCGTGTAATGACTTCAGCG
XXXXXXXXXXXXXXXXXXXXXXXXXXXXXXXXXXXXXXXXXXXXXXXXXXX

                                                        350
     .         .         .         .         .
TGCGCTTGATGCGAGATACCACACATATAGAGCAAAAAGCGCTAGCACAG
XXXXXXXXXXXXXXXXXXXXXXXXXXXXXXXXXXXXXXXXXXXXXXXXXXX
```

```
                                                            400
   .         .         .         .         .
GGGTTAGGTCAGATGCTTGAAAGCATGATTGACAACACAGTCCGTGAAAC
XXXXXXXXXXXXXXXXXXXXXXXXXXXXXXXXXXXXXXXXXXXXXXXXXXX


                                                            450
   .         .         .         .         .
GGTGGGGGCGGCAACGTCTAGAGACGCTCTCCCAAACACTGAAGCCAGTG
XXXXXXXXXXXXXXXXXXXXXXXXXXXXXXXXXXXXXXXXXXXXXXXXXXX


                                                            500
   .         .         .         .         .
GACCAGCACACTCCAAGGAAATTCCGGCACTCACCGCAGTGGAAACTGGG
XXXXXXXXXXXXXXXXXXXXXXXXXXXXXXXXXXXXXXXXXXXXXXXXXXX


                                                            550
   .         .         .         .         .
GCCACAAATCCACTAGTCCCTTCTGATACAGTGCAAACCAGACATGTTGT
XXXXXXXXXXXXXXXXXXXXXXXXXXXXXXXXXXXXXXXXXXXXXXXXXXX


                                                            600
   .         .         .         .         .
ACAACATAGGTCAAGGTCAGAGTCTAGCATAGAGTCTTTCTTCGCGCGGG   (VI)
XXXXXXXXXXXXXXXXXXXXXXXXXXXXXXXXXXXXXXXXXXXXXXXXXXX


                                                            650
   .         .         .         .         .
GTGCATGCGTGGCCATTATAACCGTGGATAACTCAGCTTCCACCAAGAAT
XXXXXXXXXXXXXXXXXXXXXXXXXXXXXXXXXXXXXXXXXXXXXXXXXXX
```

- 18 -

0195087

700

AAGGATAAGCTATTTACAGTGTGGAAGATCACTTATAAAGATACTGTCCA

XXXXXXXXXXXXXXXXXXXXXXXXXXXXXXXXXXXXXXXXXXXXXXXXXXX

750

GTTACGGAGGAAATTGGAGTTCTTCACCTATTCTAGATTTGATATGGAAT

XXXXXXXXXXXXXXXXXXXXXXXXXXXXXXXXXXXXXXXXXXXXXXXXXXX

800

TTACCTTTGTGGTTACTGCAAATTTCACTGAGACTAACAATGGGCATGCC

XXXXXXXXXXXXXXXXXXXXXXXXXXXXXXXXXXXXXXXXXXXXXXXXXXX

850

TTAAATCAAGTGTACCAAATTATGTACGTACCACCAGGCGCTCCAGTGCC

XXXXXXXXXXXXXXXXXXXXXXXXXXXXXXXXXXXXXXXXXXXXXXXXXXX

900

CGAGAAATGGGACGACTACACATGGCAAACCTCATCAAATCCATCAATCT

XXXXXXXXXXXXXXXXXXXXXXXXXXXXXXXXXXXXXXXXXXXXXXXXXXX

950

TTTACACCTACGGAACAGCTCCAGCCCGGATCTCGGTACCGTATGTTGGT

XXXXXXXXXXXXXXXXXXXXXXXXXXXXXXXXXXXXXXXXXXXXXXXXXXX

1000
ATTTCGAACGCCTATTCACACTTTTACGACGGTTTTTCCAAAGTACCACT
XXXXXXXXXXXXXXXXXXXXXXXXXXXXXXXXXXXXXXXXXXXXXXXXXXX

1050
GAAGGACCAGTCGGCAGCACTAGGTGACTCCCTCTATGGTGCAGCATCTC
XXXXXXXXXXXXXXXXXXXXXXXXXXXXXXXXXXXXXXXXXXXXXXXXXXX

1100
TAAATGACTTCGGTATTTTGGCTGTTAGAGTAGTCAATGATCACAACCCG
XXXXXXXXXXXXXXXXXXXXXXXXXXXXXXXXXXXXXXXXXXXXXXXXXXX

1150
ACCAAGGTCACCTCCAAAATCAGAGTGTATCTAAAACCCAAACACATCAG
XXXXXXXXXXXXXXXXXXXXXXXXXXXXXXXXXXXXXXXXXXXXXXXXXXX

1200
AGTCTGGTGCCCGCGTCCACCGAGGGCAGTGGCGTACTACGGCCCTGGAG
XXXXXXXXXXXXXXXXXXXXXXXXXXXXXXXXXXXXXXXXXXXXXXXXXXX

1250
TGGATTACAAGGATGGTACGCTTACACCCCTCTCCACCAAGGATCTGACC
XXXXXXXXXXXXXXXXXXXXXXXXXXXXXXXXXXXXXXXXXXXXXXXXXXX

1300

ACATATGGATTCGGACACCAAAACAAAGCGGTGTACACTGCAGGTTACAA

XXXXXXXXXXXXXXXXXXXXXXXXXXXXXXXXXXXXXXXXXXXXXXXXXXX

1350

AATTTGCAACTACCATTTGGCCACTCAGGAAGATTTGCAAAACGCAGTGA

XXXXXXXXXXXXXXXXXXXXXXXXXXXXXXXXXXXXXXXXXXXXXXXXXXX

1400

ACGTCATGTGGAATAGAGACCTCTTAGTCACAGAATCAAGAGCCCAGGGC

XXXXXXXXXXXXXXXXXXXXXXXXXXXXXXXXXXXXXXXXXXXXXXXXXXX

1450

ACCGATTCAATCGCAAGGTGCAATTGCAACGCAGGGGTGTACTACTGCGA

XXXXXXXXXXXXXXXXXXXXXXXXXXXXXXXXXXXXXXXXXXXXXXXXXXX

1500

GTCTAGAAGGAAATACTACCCAGTATCCTTCGTTGGCCCAACGTTCCAGT

XXXXXXXXXXXXXXXXXXXXXXXXXXXXXXXXXXXXXXXXXXXXXXXXXXX

1550

ACATGGAGGCTAATAACTATTACCCAGCTAGGTACCAGTCCCATATGCTC

XXXXXXXXXXXXXXXXXXXXXXXXXXXXXXXXXXXXXXXXXXXXXXXXXXX

1600

ATTGGCCATGGATTCGCATCTCCAGGGGATTGTGGTGGCATACTCAGATG

XXXXXXXXXXXXXXXXXXXXXXXXXXXXXXXXXXXXXXXXXXXXXXXXXXX

1650

TCACCACGGGGTGATAGGGATCATTACTGCTGGTGGAGAAGGGTTGGTTG

XXXXXXXXXXXXXXXXXXXXXXXXXXXXXXXXXXXXXXXXXXXXXXXXXXX

1700

CATTTACAGACATTAGAGACTTGTATGCCTACGAAGAAGAAGCCATGGAA

XXXXXXXXXXXXXXXXXXXXXXXXXXXXXXXXXXXXXXXXXXXXXXXXXXX

1750

CAAGGCATCACCAATTACATAGAGTCACTTGGGGCCCCATTTGGAAGTGG

XXXXXXXXXXXXXXXXXXXXXXXXXXXXXXXXXXXXXXXXXXXXXXXXXXX


ATTTACTCAGCTGATTGGAGACAAAATAACAGAGTT…3'

XXXXXXXXXXXXXXXXXXXXXXXXXXXXXXXXXXXX


(wherein A, G, C, T and X have the same meanings as
defined above).

Those related to Sabin type 2 are contained in the Kpn I - Pvu II fragment derived from the pVS(2)2503 plasmid [Toyoda, Haruka, et al; Journal of Molecular Biology (J. Mol. Biol.), 174, 561 (1984)].

The DNA sequence of said Kpn I - Pvu II fragment is represented by the following formula (VII):

```
                                                    50
5'···CTTGGATCAGTAATACCACATACAGACAAACCATCAACGATAGTTTCACA

CATGXXXXXXXXXXXXXXXXXXXXXXXXXXXXXXXXXXXXXXXXXXXXXXXX

                                                    100
     GAAGGTGGCTACATTAGCATGTTCTATCAAACTAGGGGTTGTTGTCCCGTT

     XXXXXXXXXXXXXXXXXXXXXXXXXXXXXXXXXXXXXXXXXXXXXXXXXX

                                                    150
     GTCCACACCCAGAAAGATGGACATCCTGGGTTTTGTGTCAGCTTGCAATG

     XXXXXXXXXXXXXXXXXXXXXXXXXXXXXXXXXXXXXXXXXXXXXXXXXX

                                                    200
     ACTTCAGTGTGCGCTTACTGCGAGATACAACACACATTAGTCAAGAGGCT

     XXXXXXXXXXXXXXXXXXXXXXXXXXXXXXXXXXXXXXXXXXXXXXXXXX
```

250

ATGCCACAAGGAATTGGTGACATGATTGAAGGGGCCGTTGAAGGGATTAC

XXXXXXXXXXXXXXXXXXXXXXXXXXXXXXXXXXXXXXXXXXXXXXXXXXX

300

TAAAAATGCATTGGTTCCCCCGACTTCCACCAATAGCCTGCCTGGACACA    (VII)

XXXXXXXXXXXXXXXXXXXXXXXXXXXXXXXXXXXXXXXXXXXXXXXXXXX

350

AGCCGAGCGGTCCAGCCCACTCCAAGGAGATACCTGCATTGACAGCCGTG

XXXXXXXXXXXXXXXXXXXXXXXXXXXXXXXXXXXXXXXXXXXXXXXXXXX

400

GAGACAGGGGCTACCAATCCGTTGGTGCCTTCGGACACCGTGCAAACGCG

XXXXXXXXXXXXXXXXXXXXXXXXXXXXXXXXXXXXXXXXXXXXXXXXXXX

450

CCATGTCATCCAGAGACGAACGCGATCAGAGTCCACGGTTGAGTCATTCT

XXXXXXXXXXXXXXXXXXXXXXXXXXXXXXXXXXXXXXXXXXXXXXXXXXX

500

TTGCAAGAGGGGCTTGCGTGGCTATCATTGAGGTGGACAATGATGCACCG

XXXXXXXXXXXXXXXXXXXXXXXXXXXXXXXXXXXXXXXXXXXXXXXXXXX

550

ACAAAGCGCGCCAGCAGATTGTTTTCGGTTTGGAAAATAACTTACAAAGA

XXXXXXXXXXXXXXXXXXXXXXXXXXXXXXXXXXXXXXXXXXXXXXXXXXX

600

TACTGTTCAACTGAGACGCAAACTGGAATTTTTCACATATTCGAGATTTG

XXXXXXXXXXXXXXXXXXXXXXXXXXXXXXXXXXXXXXXXXXXXXXXXXXX

650

ACATGGAGTTCACTTTTGTGGTCACCTCAAACTACATTGATGCAAATAAC

XXXXXXXXXXXXXXXXXXXXXXXXXXXXXXXXXXXXXXXXXXXXXXXXXXX

700

GGACATGCATTGAACCAAGTTTATCAGATAATGTATATACCACCCGGAGC

XXXXXXXXXXXXXXXXXXXXXXXXXXXXXXXXXXXXXXXXXXXXXXXXXXX

750

ACCTATCCCTGGTAAATGGAATGACTATACGTGGCAGACGTCCTCTAACC

XXXXXXXXXXXXXXXXXXXXXXXXXXXXXXXXXXXXXXXXXXXXXXXXXXX

800

CGTCGGTGTTTTACACCTATGGGGCGCCCCCAGCAAGAATATCAGTGCCC

XXXXXXXXXXXXXXXXXXXXXXXXXXXXXXXXXXXXXXXXXXXXXXXXXXX

850

TACGTGGGAATTGCTAATGCGTATTCCCACTTTTATGATGGGTTTGCAAA

XXXXXXXXXXXXXXXXXXXXXXXXXXXXXXXXXXXXXXXXXXXXXXXXXXX

900

AGTACCACTAGCGGGTCAAGCCTCAACTGAAGGCGATTCGTTGTACGGTG

XXXXXXXXXXXXXXXXXXXXXXXXXXXXXXXXXXXXXXXXXXXXXXXXXXX

950

CTGCCTCACTGAATGATTTTGGATCACTGGCTGTTCGCGTGGTAAATGAT

XXXXXXXXXXXXXXXXXXXXXXXXXXXXXXXXXXXXXXXXXXXXXXXXXXX

1000

CACAACCCCACGCGGCTCACCTCCAAGATCAGAGTGTACATGAAGCCAAA

XXXXXXXXXXXXXXXXXXXXXXXXXXXXXXXXXXXXXXXXXXXXXXXXXXX

1050

GCATGTCAGAGTCTGGTGCCCACGACCTCCACGAGCAGTCCCATACTTCG

XXXXXXXXXXXXXXXXXXXXXXXXXXXXXXXXXXXXXXXXXXXXXXXXXXX

1100

GACCAGGTGTTGATTATAAAGATGGGCTCACCCCACTACCAGAAAAGGGA

XXXXXXXXXXXXXXXXXXXXXXXXXXXXXXXXXXXXXXXXXXXXXXXXXXX

1150

TTAACGACTTATGGATTTGGACACCAAAACAAAGCTGTGTACACAG···3'

XXXXXXXXXXXXXXXXXXXXXXXXXXXXXXXXXXXXXXXXXXXXXXXXXX

(wherein A, G, C, T and X have the same meanings as defined above).

Further, those related to Sabin type 3 are contained in the Sph I - Bgl II fragment derived from the pVS(2)2503 plasmid [Toyoda, Haruka, et al; Journal of Molecular Biology (J. Mol. Biol.), 174, 561 (1984)].

The DNA sequence of said Sph I - Bgl II fragment is represented by the following formula (VIII):

50

5'···CTGGGGTTTGTGTCAGCCTGTAATGATTTCAGTGTGGGATTGCTGCGAGA

GTACXXXXXXXXXXXXXXXXXXXXXXXXXXXXXXXXXXXXXXXXXXXXXXX

100

CACCACTCACATTTCACAATCTGCGCTTCCACAGGGTATTGAAGATTTGA

XXXXXXXXXXXXXXXXXXXXXXXXXXXXXXXXXXXXXXXXXXXXXXXXXX

150

TTTCTGAAGTTGCACAGGGCGCCCTAACTTTGTCACTCCCGAAGCAACAG

XXXXXXXXXXXXXXXXXXXXXXXXXXXXXXXXXXXXXXXXXXXXXXXXXX

200

GATAGCTTACCTGATACTAAGGCCAGTGGCCCGGCGCATTCCAAGGAGGT  (VII)

XXXXXXXXXXXXXXXXXXXXXXXXXXXXXXXXXXXXXXXXXXXXXXXXXXXX

250

ACCTGCACTCACTGCAGTCGAGACTGGAGCCACCAATCCTCTGGCACCAT

XXXXXXXXXXXXXXXXXXXXXXXXXXXXXXXXXXXXXXXXXXXXXXXXXXXX

300

CCGACACAGTTCAAACGCGCCACGTAGTCCAACGACGCAGCAGGTCAGAG

XXXXXXXXXXXXXXXXXXXXXXXXXXXXXXXXXXXXXXXXXXXXXXXXXXXX

350

TCCACAATAGAATCATTCTTCGCACGCGGGGCGTGCGTCGCTATTATTGA

XXXXXXXXXXXXXXXXXXXXXXXXXXXXXXXXXXXXXXXXXXXXXXXXXXXX

400

GGTGGACAATGAACAACCAACCACCCGGGCACAGAAACTATTTGCCATGT

XXXXXXXXXXXXXXXXXXXXXXXXXXXXXXXXXXXXXXXXXXXXXXXXXXXX

450

GGCGCATTACATACAAAGATACAGTGCAGTTGCGCCGTAAGTTGGAGTTT

XXXXXXXXXXXXXXXXXXXXXXXXXXXXXXXXXXXXXXXXXXXXXXXXXXXX

- 28 -

0195087

500

TTCACATACTCTCGTTTTGACATGGAATTCACCTTCGTGGTAACCGCCAA

XXXXXXXXXXXXXXXXXXXXXXXXXXXXXXXXXXXXXXXXXXXXXXXXXXX

550

CTTCACCAACGCTAATAATGGGCATGCACTCAACCAGGTGTACCAGATAA

XXXXXXXXXXXXXXXXXXXXXXXXXXXXXXXXXXXXXXXXXXXXXXXXXXX

600

TGTACATCCCCCCAGGGGCACCCACACCAAAGTCATGGGACGACTACACT

XXXXXXXXXXXXXXXXXXXXXXXXXXXXXXXXXXXXXXXXXXXXXXXXXXX

650

TGGCAAACATCTTCCAACCCGTCCATATTTTACACCTATGGGGCTGCCCC

XXXXXXXXXXXXXXXXXXXXXXXXXXXXXXXXXXXXXXXXXXXXXXXXXXX

700

GGCGCGAATCTCAGTGCCATACGTGGGGGTTAGCCAATGCTTACTCGCACT

XXXXXXXXXXXXXXXXXXXXXXXXXXXXXXXXXXXXXXXXXXXXXXXXXXX

750

TTTACGACGGCTTCGCCAAGGTGCCATTGAAGACAGATGCCAATGACCAG

XXXXXXXXXXXXXXXXXXXXXXXXXXXXXXXXXXXXXXXXXXXXXXXXXXX

800

ATTGGTGATTCCTTGTACAGCGCCATGACAGTTGATGACTTTGGTGTATT

XXXXXXXXXXXXXXXXXXXXXXXXXXXXXXXXXXXXXXXXXXXXXXXXXXX

850

GGCAGTTCGTGTTGTCAATGATCACAACCCCACTAAAGTAACCTCCAAAG

XXXXXXXXXXXXXXXXXXXXXXXXXXXXXXXXXXXXXXXXXXXXXXXXXXX

900

TCCGCATTTACATGAAACCCAAACACGTACGTGTCTGGTGCCCTAGACCG

XXXXXXXXXXXXXXXXXXXXXXXXXXXXXXXXXXXXXXXXXXXXXXXXXXX

950

CCGCGCGCGGTACCTTATTATGGACCAGGGGTGGACTATAGGAACAACTT

XXXXXXXXXXXXXXXXXXXXXXXXXXXXXXXXXXXXXXXXXXXXXXXXXXX

1000

GGACCCCTTATCTGAGAAAGGTTTGACCACATATGGCTTTGGGCATCAGA

XXXXXXXXXXXXXXXXXXXXXXXXXXXXXXXXXXXXXXXXXXXXXXXXXXX

ATAAAGCTGTGTACACTGCTGGTTACAA···3'

XXXXXXXXXXXXXXXXXXXXXXXXXXXXXXXCTAG

(wherein A, G, C, T and X have the same meanings as
defined above).

The first cyclic double-stranded DNA of the present invention should preferably contain a temperature sensitive gene portion positioned upstream on the 5' side of the high expression promoter-operator in the opposite direction thereto for regulating its activity.

The second cyclic double-stranded DNA of the present invention should preferably contain a temperature sensitive gene portion positioned upstream on the 5' side of the high expression promoter-operator for regulating its activity.

More specifically, an example of the temperature sensitive gene portion for regulating the activity of the high expression promoter-operator is the $cI_{857}$ mutant gene of the phage $\lambda$ cI gene. The $cI_{857}$ (hereinafter called "cIts") mutant gene is a gene coding for the temeprature sensitive repressor of the $P_L$ promoter-operator.

The DNA fragment containing such a gene may be taken out directly from, for example, phage $\lambda$, but it may also be excised and used from a plasmid containing such a fragment such as the pCQV2 plasmid [Queen, C.; Journal of Molecular and Applied Genetics, 2 (1), 1 (1983)].

The DNA sequence of the cIts mutant gene portion derived from the pCQV2 plasmid may include, for example, the sequence of the following formula (IX):

50

5'···AATTCTCATGTTTGACAGCTTATCATATGAAGATTCTTGCTCAATTGTTA

XXXXXXXXXXXXXXXXXXXXXXXXXXXXXXXXXXXXXXXXXXXXXXXXXXX

100

TCAGCTATGCGCCGACCAGAACACCTTGCCGATCAGCCAAACGTCTCTTC

XXXXXXXXXXXXXXXXXXXXXXXXXXXXXXXXXXXXXXXXXXXXXXXXXXX

150

AGGCCACTGACTAGCGATAACTTTCCCCACAACGGAACAACTCTCATTGC

XXXXXXXXXXXXXXXXXXXXXXXXXXXXXXXXXXXXXXXXXXXXXXXXXXX

200

ATGGGATCATTGGGTACTGTGGGTTTAGTGGTTGTAAAAACACCTGACCG

XXXXXXXXXXXXXXXXXXXXXXXXXXXXXXXXXXXXXXXXXXXXXXXXXXX

250

CTATCCCTGATCAGTTTCTTGAAGGTAAACTCATCACCCCCAAGTCTGGC

XXXXXXXXXXXXXXXXXXXXXXXXXXXXXXXXXXXXXXXXXXXXXXXXXXX

300   (IX)

TATGCAGAAATCACCTGGCTCAACAGCCTGCTCAGGGTCAACGAGAATTA

XXXXXXXXXXXXXXXXXXXXXXXXXXXXXXXXXXXXXXXXXXXXXXXXXXX

350

ACATTCCGTCAGGAAAGCTTGGCTTGGAGCCTGTTGGTGCGGTCATGGAA

XXXXXXXXXXXXXXXXXXXXXXXXXXXXXXXXXXXXXXXXXXXXXXXXXXX

0195087

400

TTACCTTCAACCTCAAGCCAGAATGCAGAATCACTGGCTTTTTTGGTTGT

XXXXXXXXXXXXXXXXXXXXXXXXXXXXXXXXXXXXXXXXXXXXXXXXXXX

450

GCTTACCCATCTCTCCGCATCACCTTTGGTAAAGGTTCTAAGCTTAGGTG

XXXXXXXXXXXXXXXXXXXXXXXXXXXXXXXXXXXXXXXXXXXXXXXXXXX

500

AGAACATCCCTGCCTGAACATGAGAAAAAACAGGGTACTCATACTCACTT

XXXXXXXXXXXXXXXXXXXXXXXXXXXXXXXXXXXXXXXXXXXXXXXXXXX

550

CTAAGTGACGGCTGCATACTAACCGCTTCATACATCTCGTAGATTTCTCT

XXXXXXXXXXXXXXXXXXXXXXXXXXXXXXXXXXXXXXXXXXXXXXXXXXX

600

GGCGATTGAAGGGCTAAATTCTTCAACGCTAACTTTGAGAATTTTTGCAA

XXXXXXXXXXXXXXXXXXXXXXXXXXXXXXXXXXXXXXXXXXXXXXXXXXX

650

GCAATGCGGCGTTATAAGCATTTAATGCATTGATGCCATTAAATAAAGCA

XXXXXXXXXXXXXXXXXXXXXXXXXXXXXXXXXXXXXXXXXXXXXXXXXXXX

700

CCAACGCCTGACTGCCCCATCCCCATCTTGTCTGCGACAGATTCCTGGGA

XXXXXXXXXXXXXXXXXXXXXXXXXXXXXXXXXXXXXXXXXXXXXXXXXXX


750

TAAGCCAAGTTCATTTTTCTTTTTTTCATAAATTGCTTTAAGGCGACGTG

XXXXXXXXXXXXXXXXXXXXXXXXXXXXXXXXXXXXXXXXXXXXXXXXXXX


800

CGTCCTCAAGCTGCTCTTGTGTTAATGGTTTCTTTTTTGTGCTCATACGT

XXXXXXXXXXXXXXXXXXXXXXXXXXXXXXXXXXXXXXXXXXXXXXXXXXX


850

TAAATCTATCACCGCAAGGGATAAATATCTAACACCGTGCGTGTTGACTA

XXXXXXXXXXXXXXXXXXXXXXXXXXXXXXXXXXXXXXXXXXXXXXXXXXX


900

TTTTACCTCTGGCGGTGATAATGGTTGCATGTACTAAGGAGGTTGTATG ··

XXXXXXXXXXXXXXXXXXXXXXXXXXXXXXXXXXXXXXXXXXXXXXXXXXXC


··· ··· 3'

TAG


(wherein A, G, C, T and X have the same meanings as defined above).

The cyclic double-stranded DNA of the present invention should preferably contain a portion coding for ampicillin resistance downstream of the temperature sensitive gene portion, and said portion should more preferably be derived from the pBR322 plasmid.

The first cyclic double-stranded DNA can be prepared, for example, as follows.

The pCQV2 plasmid is digested with the restriction endonuclease Cla I and the protruded end digested with S1 nuclease to make it a blunt end, which is cyclized again with T4DNA ligase, thereby extinguishing the Cla I site to obtain the pHT 1 plasmid. The cyclic DNA obtained was transformed into, for example, Escherichia coli HB 101 strain following the method of Cohen et al [Proceeding National Academic Science USA (Proc. Natl. Acad. Sci. USA), 69, 2110 (1972)], and the pHT1 plasmid containing strains are screened with ampicillin resistance as the marker and amplified. From the transformed strain, the pHT 1 plasmid is isolated. Between the Bam HI site and the Eco RI site of the plasmid, there is contained the temperature sensitive gene cIts which is the repressor of the $P_R$ and $P_L$ promoters of phage $\lambda$.

By digesting the pHT1 plasmid with Eco RI and Bam HI, the fragment containing cIts is isolated. This fragment comprises the DNA sequence shown by the above formula (IX). The $\frac{CG}{GC}$ base pairs which existed at the Cla I site of the corresponding fragment of pCQV 2 (which existed between the $\frac{T}{A}$ base pair at the 26 th and the $\frac{A}{T}$ base pair at the 27 th in the above formula (IX)) has been removed.

On the other hand, the pKC 30 plasmid is digested with Eco RI and Bgl II to separate a long fragment therefrom.

This fragment has one Bam HI site, and the DNA sequence from the Eco RI end to the Bam HI site corresponds to the DNA sequence anticlockwise from the Eco RI site of the pBR322 to the Bam HI site on the restriction endonuclease cleaved map.  In this sequence, the portion coding for ampicillin resistance and the initiation point for duplication in E. coli are contained.  There exists also one Hpa I site between the Bam HI site and the Bgl II end, and the $P_L$ promoter-operator exists between the Bgl II end and the Hpa I site.  The DNA sequence between the Bgl II end and the Hpa I site is shown by the above formula (I).

The both fragments as obtained above are ligated with $T_4$DNA ligase and cyclized to construct the pHT 2 plasmid.  The plasmid is cloned similarly as the pHT 1 plasmid to be amplified.

The pYT 3 plasmid is digested with the restriction endonuclease Sst II, the C-230 fragment containing the portion coding for metapyrocatechase is isolated and the 3'-protruded end is digested with $T_4$DNA polymerase to be made a blunt end.

The pHT 2 plasmid is subjected to ring opening by digestion with the restriction endonuclease Hpa I and then the C-230 fragment with the blunt end is inserted thereinto, followed by ring closure, to construct the pHT 3 plasmid.  E. coli is transformed with this plasmid and, with the use of ampicillin resistance as the marker, the colony colored in yelow with an aqueous catechol solution after elevation of the culture temperature to about 42 °C in the course of cultivation is collected, followed by cloning and amplification of the plasmid.

In place of the C-230 fragment from the pYT 3 plasmid, the pSLMK 1 plasmid (of the transformants having this plasmid, Escherichia coli HB 101/pSLMK 1 is deposited at FERM as the FERM P-7816, Escherichia coli W 3110/pSLMK 1 as the FERM P-7617, Escherichia coli RR 1/pSLMK 1 as the FERM P-7618 and Escherichia coli RB 791/pSLMK 1 as the FERM P-7619, respectively) or the pTCCM 1 plasmid (of the transformants having this plasmid, Escherichia coli RB 791/pTCCM is deposited at FERM on August 17, 1984 as FERM P-77815, and transferred to the international deposition based on the Budapest Treaty on August 8, 1985, and given the number of FERM BP-862) may be digested with Eco RI, and the resultant C-230 fragment filled at both protruded ends with dNTP and inserted into the plamid pHT 2 to prepare the pHT 3 plasmid.

The method for preparation of the pSLMK 1 plasmid is described in detail in Japanese Patent Application No. 97106/1984.

Next, the pHT 3 plasmid is digested with Bam HI, the protruded ends are repaired with $T_4$DNA polymerase or Klenow fragment to be made blunt ends, and further digested partially with Pvu II, followed again by cyclization with $T_4$DNA ligase to obtain the pHT 31 plasmid. At the ligated portion, the Bam HI portion is regenerated. The pHT 31 plasmid is digested with Sal I, the protruded portions are repaired with $T_4$DNA ligase or Klenow fragment to make them blunt ends, and further digested with Pvu II, followed again by cyclization with $T_4$DNA ligase to construct the pHT 311 plasmid. At the ligated portion, the Sal I is regenerated. The operations of the pHT 3 plasmid et seq are operations for making an appropriate restriction endonuclease site at the DNA portion coding for the N-terminus portion of metapyrocatechase, and it is also possible to attempt insertion of the poliovirus cDNA fratment through utilization of the Pvu II site in the metapyrocatechase gene by digesting the pHT 3 plasmid partially with Pvu II.

Next, the pHT 311 plasmid is digested with Sal I, the protruded ends are repaired with $T_4$DNA polymerase or Klenow fragment to be made blunt ends to obtain a DNA fragment. On the other hand, a DNA fragment is prepared containing the VP1 gene from the cDNA derived from poliovirus.

For example, in Sabin type 1, the pBB 2 plasmid is digested with Taq I, the protruded ends are repaired with $T_4$DNA polymerase and a DNA fragment containing VP1 is isolated. Said DNA fragment and the DNA fragment obtained from the pHT 311 plasmid as described above are subjected to ligating cyclization with $T_4$DNA ligase to construct the pHTP 31 plasmid.

The pBB 2 plasmid is digested with Bam HI and Hin cII, the protruded ends are repaired with $T_4$DNA polymerase, and the DNA fragment containing VP1 is isolated. Said DNA fragment and the DNA fragment obtained from the pHT 311 plasmid as described above asre subjected to ligating cyclization with $T_4$DNA ligase to construct the pYTP 100 plasmid. The pHTP plasmid is digested with Kpn I to prepare a DNA fragment.

The pBB 2 plasmid is digested with Kpn I to separate a DNA fragment containing the carboxylic terminus (hereinafter called "C-terminus") of VP1. Said fragment and the DNA fragment obtained by digestion of the pHTP 31 plasmid with Kpn I are subjected to ligating cyclization with $T_4$DNA ligase to construct the pYTP 11 plasmid.

As for Sabin type 2, for example, the pVS(2)2503 plasmid is digested with Kpn I and Pvu II, the protruded ends are repaired with $T_4$DNA to be made blunt ends, and the DNA fragment containing VP1 is isolated.

Said DNA fragment and the DNA fragment obtained from the pHT 311 plasmid as described above are subjected to ligating cyclization to construct the pYTP 200 plasmid.

As for Sabin type 3, for example, the pVS(3)2603 plasmid is digested with Sph I and Bgl II, the protruded ends are repaired with $T_4$DNA polymeraze or Klenow fragment to be made blunt ends, and the DNA fragment containing VP1 is isolated. Said DNA fragment and the DNA fragment obtained from the pHT 311 plasmid as described above are subjected to ligating cyclization to construct the pYTP 300-1 plasmid. This does not coincide in frame of amino acids of a part of the metapyrocatechase gene and the DNA derived from poliovirus. Accordingly, for coincidence in frame, the pYTP 300-1 plasmid is digested with Sal I, the protruded ends digested with S1 nuclease, followed by recylization with $T_4$DNA ligase to construct the pYTP 300 plasmid.

The second cyclic double-stranded DNA of the present invention can be prepared, for example, as follows.

First, for making the N-terminus of the poliovirus VP1 protein in the non-fused form, the vector pYTU3 for expression (deposited at FERM as Escherichia coli HB101/pYTU3 FERM P-7982) can be utilized. The pYTU plasmid is digested with Sal I, the protruded ends are digested with S1 nuclease to be made blunt ends, whereinto the DNA fragment coding for the VP1 antigen region of poliovirus Sabin type 1, Sabin type 2 or Sabin type 3 made to have blunt ends is inserted. In the case of Sabin type 1, for example, the fused type expression plasmid pHTP31 plasmid or the pYTP11 plasmid can be used.

The pHTP31 plasmid and the pYTP11 plasmid are deposited at American Type Culture Collection (ATCC) as Escherichia coli C 600/pHTP31 and Escherichia coli C 600/pYTP11, respectively, and attached with the deposition numbers of ATCC 39976 and ATCC 39972, respectively.

When using the pHTP31 plasmid, this is digested with the restriction endonuclease Sal I to obtain a DNA fragment of 713 base pairs. The DNA fragment may be blunted at the ends with $T_4$DNA polymerase and insereted into the Sal I site of the blunt end of pYTU3 with the use of $T_4$DNA ligase, but without blunting of the ends, the Sal I digested DNA fragment of the pYTU3 plasmid and the DNA fragment of Sal I 713 base pairs containing the VP1 protein antigen site can be cyclized again with the use of $T_4$DNA ligase to obtain the pYTP102.

Next, the operation of making the C end non-fused is conducted. The pYTP102 plasmid is digested with the restriction endonuclease Kpn I and then blunted the ends with T4DNA polymerase.

The commercially available oligonucleotide of 16 base pairs having translation termination codon TAA in three frames (hereinafter called "translation termination unit") represented by the following formula (X):

        5'···GCTTAATTAATTAAGC···3'
        3'···CGAATTAATTAATTCG···5'

        (wherein A, G, C and T have the same meanings as
        defiend above),
is phosphorylated at the 5'-end with $T_4$ polynucleotide kinase, and this was subjected to ligating cyclization with $T_4$DNA ligase together with the DNA fragment obtained by digestion of the pYTP 102 plasmid with Kpn I, followed by blunting of the ends with $T_4$DNA polymerase, to obtain the pYTP102K plasmid.

On the other hand, non-fused N-terminus and C-terminus can be also constructed at the same time. The pYTP11 plasmid is digested with Pst I, followed by blunting of the ends with T$_4$DNA polymerase. Said DNA fragment is ligated with the translation termination unit with T$_4$DNA ligase. Further, after digestion of the restriction endonuclease Sal I followed by agarose gel electrophoresis, the DNA fragment of 1050 base pairs containing the region coding for the VP1 protein antigen site is recovered.

Double digestion of the pYTU3 plasmid with the restriction endonucleases Sal I and Nru I gives a DNA fragment of 4000 base pairs, which is subjected together with the above DNA fragment of 1050 base pairs to ligating cyclization with T$_4$DNA ligase to obain the pYTP102P plasmid.

As for Sabin type 2, for example, after digestion of the fused type expressed plasmid pYTP200 with the restriction endonuclease Sal I followed by agarose gel electrophoresis, the DNA fragment of about 1160 base pairs containing the region coding for the VP1 protein antigen site is recovered. Said fragment and the DNA fragment obtained by digestion with the pYTU3 plasmid are subjected to ligating cyclization to construct the pYTP201.

The pYTP201 plasmid is digested with the restriction endonuclease Nar I and blunted the ends with T$_4$DNA polymerase.

Said DNA fragment and the 5'-end phosphorylated product obtained by treating the translation termination unit with T$_4$polynucleotide kinase are subjected to ligating cyclization with T$_4$DNA ligase to construct the pYTP201N plasmid.

The pYTP plasmid is deposited at ATCC as Escherichia coli 600/pYTP200, and attached with the deposition number of ATCC 39977.

As for Sabin type 3, for example, after digestion of the fused type expressed plasmid pYTP300 with the restriction endonuclease Pst I, it is blunted the ends with $T_4DNA$ polymerase and then digested with the restriction endo- nuclease Bam HI, followed by agarose gel electrophoresis, to obtain a DNA fragment of about 1500 base pairs.

On the other hand, the pYTU3 plasmid is digested with the restriction endonuclease Sal I, followed by blunting the ends with S1 nuclease. Subsequently, after digestion with the restriction endonucelase with Bam HI followed by agarose gel electrophoresis, a DNA fragment of about 3800 base pairs is recovered. Said DNA fragment and the above fragment of about 1500 base pairs are subjected to ligating cyclization with $T_4DNA$ ligase to obtain a pYTP301 plasmid.

The pYTP301 plasmid is digested with the restriction endonuclease Kpn I, blunted the ends with $T_4DNA$ polyme- rase, and then said DNA fragment and the 5'-end phosphorylated product obtained by treatment of the translation termination unit with $T_4$polynucleotide kinase were subjected to ligating cyclization with $T_4DNA$ ligase to obtain the pYTP301K plasmid.

The pYTP300 plasmid is deposited at ATCC as Escherichia coli C 600/pYTP 300, and is attached with the deposition number of ATCC 39973.

Of the plasmids as prepared above, the pYTP102K plasmid, the pYTP102P plasmid and the pYTP301K plasmid code only methionine coded by ATG at the N-terminus and only 1 to 2 amino acids coded by the translation termination unit at the C-terminus other than the polypeptide derived from the poliovirus Sabin type, while the pYTP201N plasmid codes methionine coded by ATG and one or two amino acids for coding the restriction endonuclease cleavage site at the N-terminus and only one amino acid coded by the translation termination unit at the C-terminus other than the polypeptide derived from the poliovirus Sabin type.

In the above operations, for digestion with respective restriction endonucleases, ligation with $T_4$DNA ligase, screening with the use of ampicillin resistance and amplification of respective plasmids, all conventional methods can be used. For purification by separation of respective DNA fragments and plasmids, conventional techniques such as extraction with phenol, precipitation with ethanol agarose gel electrophoresis and subsequent extraction techniques, separation by liquid chromatogrpahy, etc. can be utilized.

By transformation of a bacterium of the genus Escherichia with the use of the cyclic double-stranded DNA of the present invention as prepared above, a microorganism capable of producing a protein having the antigenic determinant of the capsid protein VP1 (hereinafter the fused type is called "VP1 fused protein", and the non-fused type "VP1 non-fused protein") can be obtained. As the bacterium of the genus Escherichia, it is preferable to use Escherichia coli.

Typical microorganism strains capable of producing VP1 fused protein as obtained above are deposited at ATCC, and attached with the following deposition numbers.

|  | Deposition No. |
|---|---|
| Escherichia coli C 600/pHTP31 | ATCC 39976 |
| Escherichia coli C 600/pYTP11 | ATCC 39972 |

Escherichia coli C 600/pYTP100  ATCC 39975
Escherichia coli C 600/pYTP200  ATCC 39977
Escherichia coli C 600/pYTP300  ATCC 39973
Escherichia coli C 600/pSLMK1  ATCC 39974

Typical microorganism strains capable of producing VP1 non-fused proteins are as follows:

Escherichia coli C 600/pHTP102P
Escherichia coli C 600/pYTP102K
Escherichia coli C 600/pYTP201N
Escherichia coli C 600/pHTP301K

These microorganisms were applied to be deposited at FERM, but rejected for the reason of corresponding to P2 level, but they were deposited at Deutsche Sammlung von Mikroorganismen (DSM) on September 12, 1985, and attached with the following deposition numbers.

|  | Deposition No. |
|---|---|
| Escherichia coli C 600/pHTP102P | DSM 3470 |
| Escherichia coli C 600/pYTP102K | DSM 3469 |
| Escherichia coli C 600/pYTP201N | DSM 3472 |
| Escherichia coli C 600/pHTP301K | DSM 3471 |

The present invention also provides a process for preparing a poliovirus vaccine, which compriese culturing the above transformed strain in a nutrient medium and harvesting the fused protein or non-fused protein accumulated in the microorganism cells.

As the nutrient medium to be used in the present invention, conventional media such as LB medium, LB agar medium, SB medium, etc. may be used. These media may be added optionally with a chemical reagent such as ampicillin.

0195087

In the process of the present invention, the transformed strain can be cultured according to the conventional method such as culturing on a solid medium, liquid aeration cultivation, etc. The culturing conditions such as culture temperature, liquid properties of the medium may be the same as those for, for example, Escherichia coli C600, Escherichia coli W3110, Escherichia coli RB791, Escherichia coli RR1, etc. The cultivation time may be generally from several hours to one day.

The transformant to be used in the process of the present invention is inactivated in the cIts protein which is the repressor of the high expression promoter-operator, such as the $P_L$ promoter-operator, by making the cultivation temperature from an ordinary temperature of 30 °C to about 42 °C in the course of or after growth, whereby said promoter-operator can be activated and the gene portion coding for the VP1 fused protain or the VP1 non-fused protein under its control can be potently transcribed and expressed.

The grown microorganisms are collected in a conventional manner, and the cells are destroyed by sonication or the conventional cell treating method generally employed with enzymes or surfactants, optionally extracted with an aqueous solution containing a highly concentrated modifier such as urea or guanidine chloride, followed by restoration of the activity by dilution, etc., to give the desired VP1 fused protein or VP1 non-fused protein. Also, by dialysing this extract against Tris-hydrochloride buffer (hereinafter called "Tris-HCl") or phosphate buffer, the desired product can be obtained as a soluble protein.

[Brief description of the drawings]

Fig. 1, Fig. 2, Fig. 3, Fig. 4, Fig. 5, Fig. 6, Fig. 7 and Fig. 8 are diagrams illustrating preparation examples of the cyclic double-stranded DNA of the present invention.

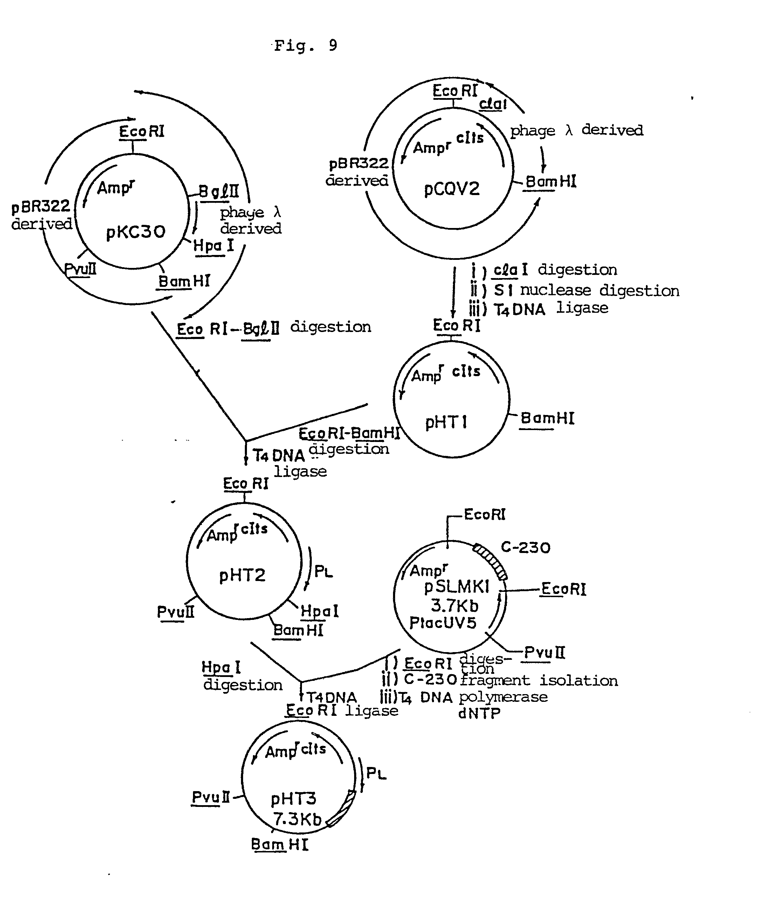

Fig. 9 is a diagram illustrating a preparation example of the pHT3 plasmid.

[Best modes for practicing the invention]

Referring now to the Examples and Preparation examples, the present invention is described in more detail, but the present invention is not limited thereto within the spirit of the invention.

The buffers and the media employed in the following description had the compositions as shown below.

TE buffer:
    10mM Tris-HCl
     1mM EDTA
    pH 7.6 or 8.0.

Stopping solution:
    1 % Sodium dodecyl sulfate,
    0.1 % Bromophenol blue,
    100 mM Disodium ethylenediaminetetraacetate (hereinafter called "EDTA"), and
    50 % Glycerine are contained (produced by Bethesda Research Laboratories Incorproated).

LB amp medium:
| | |
|---|---|
| NaCl | 5 g |
| Peptone (Bactotryptone, trade mark | 5 g |

| Yeast extract | 5 g |
| Ampicillin | 25 mg |
| Water | 1 liter |

LB amp agar medium:

In addition to the respective components of the above LB amp medium, 15 g of agar is contained.

For screening medium, LB amp agar medium is used and LB amp medium is used for the culture broth for amplification.

Preparation example:

[I] Preparation of pHT 1

7 µg of the PCQV2 plasmid was incubated with 50 U of Cla I in 50 µl of a reaction medium (6 mM Tris-HCl pH 7.9, 6 mM $MgCl_2$, 50 mM NaCl) at 37 °C for 2 hours. After phenol extraction and ethanol precipitation, incubation was carried out with 10 U of S1 nuclease in 50 µl of a reaction medium (0.2 M NaCl, 50 mM sodium acetate pH 4.5, 1 mM $ZnSO_4$, 5 % glycerine) at 37 °C for 2 minutes. After the incubation, phenol extraction and ethanol precipitation were conducted, followed by incubation with the use of 2.5 U of $T_4$ DNA ligase in 50 µl of a reaction medium (66 mM Tris-HCl pH 7.6, 6.6 mM $MgCl_2$, 1 mM ATP, 5 mM DTT) at 15 °C for 17 hours. After the incubation, incubation was further carried out with addition of 6 µl of 50 mM NaCl and 20 U of Cla I at 37 °C for 2 hours, followed further by heat treatment at 70 °C for 5 minutes for inactivation of the enzyme. After left to cool, the Escherichia coli HB 101 strain was transformed by use of this solution, the colony having the pHT 1 plasmid was screened with ampicillin resistance as the marker, and the plasmid was isolated following a conventional means.

## [II] Isolation of the cIts gene

10 µg of the pHT 1 plasmid was incubated with 100 U of Bam HI in 50 µl of a reaction medium (10 mM Tris-HCl pH 8.0, 7 mM $MgCl_2$, 0.1 M NaCl, 2 mM β-mercaptoethanol) at 30 °C for 3 hours. After the incubation, the mixture was subjected to heat treatment at 70 °C for 5 minutes, left to cool and then incubated with addition of 10 µl (100 U) of Eco RI, 10 µl of a buffer (1M Tris-HCl pH 7.5, 70 mM $MgCl_2$, 500 mM NaCl, 90 mM β-mercaptoethanol) and 25 µl of water to a total amount of 100 µl at 37 °C for 2 hours. After the incubation, the mixture was heated at 70 °C for 5 minutes and left to cool. To 30 µl of this solution was added 5 µl of a stopping solution, and electrophoresis was conducted (50 mA, 30 minutes) on 0.7 % agarose gel for separation. The fragment of 400 base pairs containing the cIts gene was excised and, after purification, dissolved in 20 µl of water.

## [III] Preparation of pHT 2

8 µg of the pKC 30 plasmid was incubated with 80 U of Bgl II in 60 µl of a reaction medium (10 mM Tris-HCl pH 7.5, 7 mM $MgCl_2$, 60 mM NaCl, 7 mM β-mercaptoethanol) at 37 °C for 90 minutes. After the incubation, phenol extraction, ethanol precipitation were conducted and the precipitate was dissolved in 10 µl of water. To the solution were added 10 µl of a buffer (1M Tris-HCl pH 7.5, 70 mM $MgCl_2$, 500 mM NaCl, 90 mM β-mercaptoethanol), 15 µl (80 U) of Eco RI and water to make up a total volume of 100 µl, and incubation was carried out at 37 °C for 90 minutes. After the incubation, phenol extraction, ethanol precipitation were conducted and the precipitate was dissolved in 20 µl of water. To 1 µl of this solution were added 5 µl of the solution prepared in [II], 2 µl of a buffer (660 mM Tris-HCl pH 7.6, 66 mM $MgCl_2$, 50 mM DTT, 10 mM ATP), 7 µl of water and 1 µl (2.8 U) of $T_4$DNA ligase, followed by incubation at 15 °C for 16 hours. After the reaction, 90 µl of water was added to 10 µl of this solution, and the Escherichia coli HB 101 strain was transformed similarly as in the case of the pHT1 plasmid. The colony having the pHT 2 plasmid was screened with ampicillin resistance as the marker, and the plasmid was isolated.

[IV] Preparation of metapyrocatechase gene

33 µg of the pSLMK 1 plasmid was incubated with Eco RI (180 U) in 50 µl of a reaction medium (0.1 M Tris-HCl pH 7.5, 7 mM $MgCl_2$, 50 mM NaCl, 9 mM β-mercaptoethanol) at 37 °C for 3 hours. After the incubation, the reaction mixture was heated at 70 °C for 5 minutes. After left to cool, the reaction was added with 8 µl of a stopping solution and subjected to electrophoresis (150 mA, 2.5 hours) on 0.7 % agarose gel for separation. The DNA fragment of 1200 base pairs containing the metapyrocatechase gene was excised, purified in a conventional manner and dissolved in 20 µl of water. The fragment was blunted the ends by incubation with 5 U of the DNA polymerase I Klenow fragment in 50 µl of a reaction medium (50 mM Tris-HCl pH 7.2, 10 mM $MgCl_2$, 0.1 mM DTT, 0.04 mM dNTP) containing 18 µl of this solution at 25 °C for 30 minutes. After the incubation, phenol extraction and chloroform extraction were conducted, followed by ethanol precipitation, and the precipitate was dissolved in 10 µl of water.

[V] Preparation of pHT 3

5.4 g of the plasmid pHT 2 obtained in [III] was incubated with 60 U of Hpa I in 50 µl o f a reaction medium (10 mM Tris-HCl pH 7.5, 7 mM $MgCl_2$, 0.1 M KCl, 7 mM β-

mercaptoethanol) at 37 $^{\circ}$C for 3 hours.  After the incubation, phenol extraction and ethanol precipitation were conducted, and the precipitate was dissolved in 20 µl of water.  To 15 µl of this solution were added 8 µl of the DNA fragment solution obtained in [IV], 5 µl of a buffer (660 mM Tris-HCl pH 7.6, 66 mM MgCl$_2$, 50 mM DTT, 10 mM ATP), 7 µl of water and 15 µl (42 U) of T$_4$DNA ligase, followed by incubation at 12 $^{\circ}$C for 18 hours.  Then, after phenol extraction and ethanol precipitation, the precipitate was dissolved in 20 µl of water.  To this solution were added 3 µl of a buffer (100 mM Tris-HCl pH 7.5, 70 mM MgCl$_2$, 1 M KCl, 70 mM β-mercaptoethanol), 9 µl of water and 8 µl (48 U) of <u>Hpa</u> I, and incubation was carried out at 37 $^{\circ}$C for 3 hours, followed by heat treatment at 70 $^{\circ}$C for 5 minutes.  After left to cool, with the use of this solution, the <u>Escherichia</u> <u>coli</u> HB 101 strain was transformed similarly as in the case of the pHT 1 plasmid, and the microorganism strain having the pHT plasmid was screened with ampicillin resistance and the fact that the cells are tinted in yellow by spraying of 5 % aqueous catechol solution after elevation of the cultivation temperature from 30 $^{\circ}$C to 42 $^{\circ}$C as markers. Further, the microorganism cells were cultured in a culture broth for amplification according to conventional means and purified to obtain 2 mg of the pHT 3 plasmid.

The pHT 3 plasmid obtained as described above was introduced into various species of bacteria of the genus <u>Escherichia</u>, deposited at FERM on August 17, 1984, and attached with the deposition numbers as shown below:

<u>Escherichia</u> <u>coli</u> HB101/pHT3 FERM P-7776
<u>Escherichia</u> <u>coli</u> C600/pHT3  FERM P-7777
<u>Escherichia</u> <u>coli</u> W3110/pHT3 FERM P-7778

[VI] Preparation of pHT311

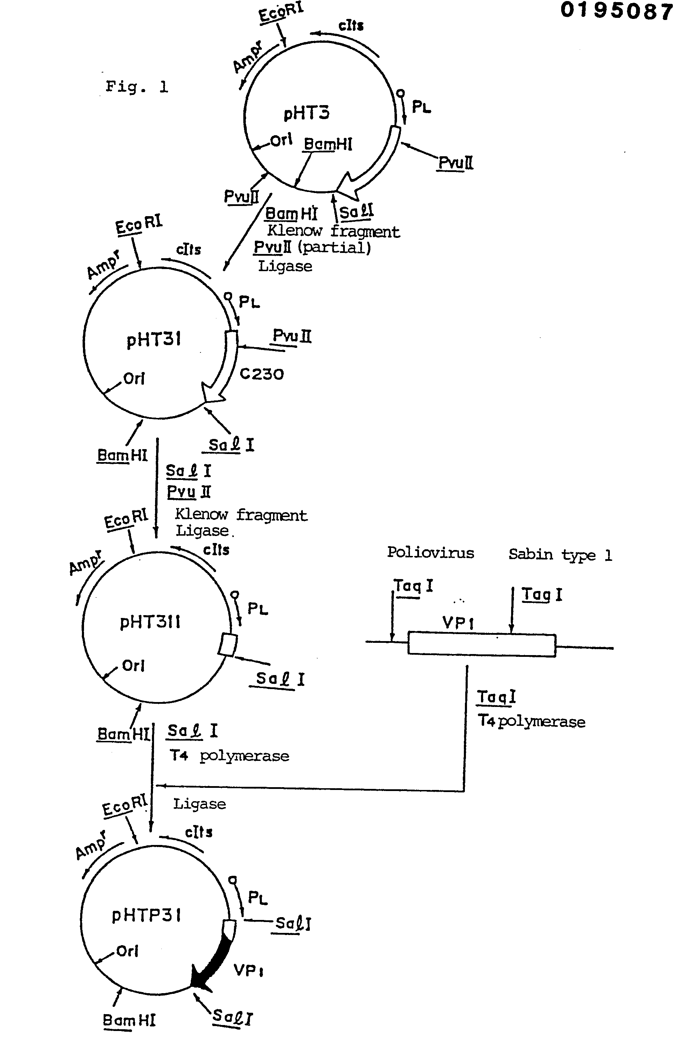

5 μg of the pHT 3 plasmid obtained in [V] and 15 U of <u>Bam</u> HI were incuabed in 20 μl of a reaction medium (10 mM Tris-HCl pH 8.0, 7 mM $MgCl_2$, 100 mM NaCl, 2 mM β-mercaptoethanol) for 3 hours. After ethanol precipitation, the precipitate was dissolved in 20 μl of a reaction medium (50 mM Tris-HCl pH 7.2, 10 mM $MgCl_2$, 0.1 mM DTT, 80 μM dNTP) and incubated with addition of 1 U of Klenow fragment at 22 °C for 0.5 hours. After deproteinization with phenol, ethanol precipitation was effected. Further, the precipitate was dissolved in 20 μl of a reaction medium (10 mM Tris-HCl pH 7.5, 7 mM $MgCl_2$, 60 mM NaCl, 7 mM β-mercaptoethanol) and incubated with 20 U of <u>Pvu</u> II at 37 °C for 3 hours. After ethanol precipitation, the precipitate was dissolved in 20 μl of a reaction medium (66 mM Tris-HCl pH 7.6, 6.6 mM $MgCl_2$, 10 mM dithiothreitol, 1 mM ATP) and incubated with addition of 2.8 U of $T_4$DNA ligase at 15 °C for 20 hours. By use of this reaction product, <u>Echerichia coli</u>HB 101 strain was transformed. From among the ampicillin resistant transformants, the microorganism having the pHT 31 plasmid was isolated. Further, by use of a conventional method, the pHT31 plasmid was obtained. 5 μg of the pHT plasmid was incubated together with 15 U of <u>Sal</u> I in 20 μl of a reaction medium (10mM Tris-HCl pH 7.5, 7mM $MgCl_2$, 150mM NaCl, 0.2mM EDTA, 7mM β-mercaptoethanol) at 37 °C for 3 hours. After ethanol precipitation, the precipitate was dissolved in 20 μl of a Klenow fragment reaction solution and incubated together with 1 U of Klenow fragment at 22 °C for 0.5 hours. After ethanol precipitation, the precipitate was dissolved in 20 μl of a <u>Pvu</u> II reaction solution and incubated with 20 U of <u>Pvu</u> II at 37 °C for 3 hours. After ethanol precipitation, the precipitate was dissolved in 20 μl of a $T_4$DNA ligase reaction solution and incubated together with 2.8 U of $T_4$DNA ligase at 15 °C for 20 hours.  By use of this reaction product, Escherichia coli HB 101 strain was transformed.  From among the ampicillin resistant transformants, the pHT311 plasmid as shown in Fig. 1 was isolated.  This plasmid has one Sal I site, and a fused gene with the metapyrocatechase gene can be prepared by inserting an extraneous gene here.

Example 1  Preparation of pHTP31

After 20 µg of the pBB2 plasmid [Nomoto, Akio (Nomoto, A.), et al.; Proceeding National Academic Science USA (Proc. Natl. Acad. Sci. USA), 79, 5793 (1982)] was incubated with 20U of Taq I in 20 µl of a buffer (10 mM Tris-HCl pH 7.5, 7 mM $MgCl_2$, 100 mM NaCl, 7 mM β-mercaptoethanol) at 60 °C for 2 hours, 4 % polyacrylamide electrophoresis was carried out.  The DNA fragment of about 700 base pairs was recovered following the method of Maxam and Gilbert [Proceeding National Academic Science USA (Proc. Natl. Acad. Sci), 74, 560 (1977)]. This was incubated with 2.5 U of $T_4$DNA polymerase in 20 µl of a buffer (67 mM Tris-HCl pH 8.8, 6.7 mM $MgCl_2$, 10 mM β-mercaptoethanol, 6.7 µM EDTA, 16.6 mM $(NH_4)_2SO_4$, 330 µM dCTP, dATP, dGTP, dTTP) at 37 °C for 15 minutes. After phenol treatment, ethanol precipitation was effected, and the precipitate was dissolved in 10 µl of 10 mM Tris-HCl pH 7.5, 1 mM EDTA solution.  This solution contained 0.08 µg of DNA per 1 µl.  This DNA fragment is of the 713 base pairs (2373 - 3085) containing the DNA region coding for the capsid protein VP1, of the cDNA obtained from the poliovirus Sabin 1 strain.

On the other hand, 10 µg of the pHT311 plasmid was incubated with 10 U of Sal I in 20 µl of a buffer (10 mM Tris-HCl pH 7.5, 7 mM $MgCl_2$, 150 mM NaCl, 0.2 mM EDTA, 7 mM β-mercaptoethanol) at 37 °C for 2 hours.  After ethanol precipitation, the precipitate was incubated with 2.5 U of $T_4$DNA polymerase in 20 µl of a buffer (67 mM Tris-HCl pH 8.8, 6.7 mM $MgCl_2$, 10 mM β-mercaptoethanol, 6.7 mM EDTA, 16.6 mM $(NH_4)_2SO_4$, 330 µM dCTP, dATP, dGTP, dTTP) at 37 °C for 15 minutes. After phenol treatment, ethanol precipitation was conducted. The precipitate was incubated with 1 U of alkaline phosphatase (bovine intestine) in 20 µl of a buffer (50 mM Tris-HCl pH 9.0, 1 mM $MgCl_2$, 0.1 mM $ZnCl_2$, 1 mM spermidine) at 37 °C for 30 minutes. After phenol treatment, ethanol precipitation was conducted. The precipitate was dissolved in 20 µl of 10 mM Tris-HCl pH 7.5, 1 mM EDTA solution. The solution contained 0.4 µg of DNA per 1 µl.

1 µg of this DNA together with 0.4 µg of the DNA fragment containing the DNA region coding for the poliovirus Sabin type 1 VP1 as described above were incubated with 2.8 U of $T_4$DNA ligase in 20 µl of a buffer (66 mM Tris-HCl pH 7.6, 6.6 mM $MgCl_2$, 10 mM DTT, 1 mM ATP) at 15 °C for 15 hours. By use of this reaction product, Escherichia coli HB 101 strain was transformed, and the pHTP31 plasmid shown in Fig. 1 was isolated from among the ampicillin resistant strains.

The cDNA of the poliovirus Sabin type 1 contained in this plasmid corresponds to the 713 base pairs from the 2373th base to the 3085th base shown in the literature [Nomoto, Akio (Nomoto, A.), et al.; Proceeding National Academic Science USA (Proc. Natl. Acad. Sci. USA), 79, 5793 (1982)].

Example 2   Preparation of pYTP 11

15 µg of the pBB2 plasmid was incubated with Kpn I in 20 µl of a buffer (10 mM Tris-HCl pH 7.5, 7 mM $MgCl_2$, 7 mM β-mercaptoethanol) at 37 °C for 3 hours, and then subjected to 1.2 % agarose electrophoresis. The DNA fragment of about 600 base pairs was recovered following the method of Dretzen et al [Analytical Biochemistry (Anal. Biochem.), 112, 295 (1981)]. This was dissolved in 20 µl of 10 mM Tris-HCl pH 7.5, 1 mM EDTA solution. This solution contained 0.03 µg of DNA per 1 µl.

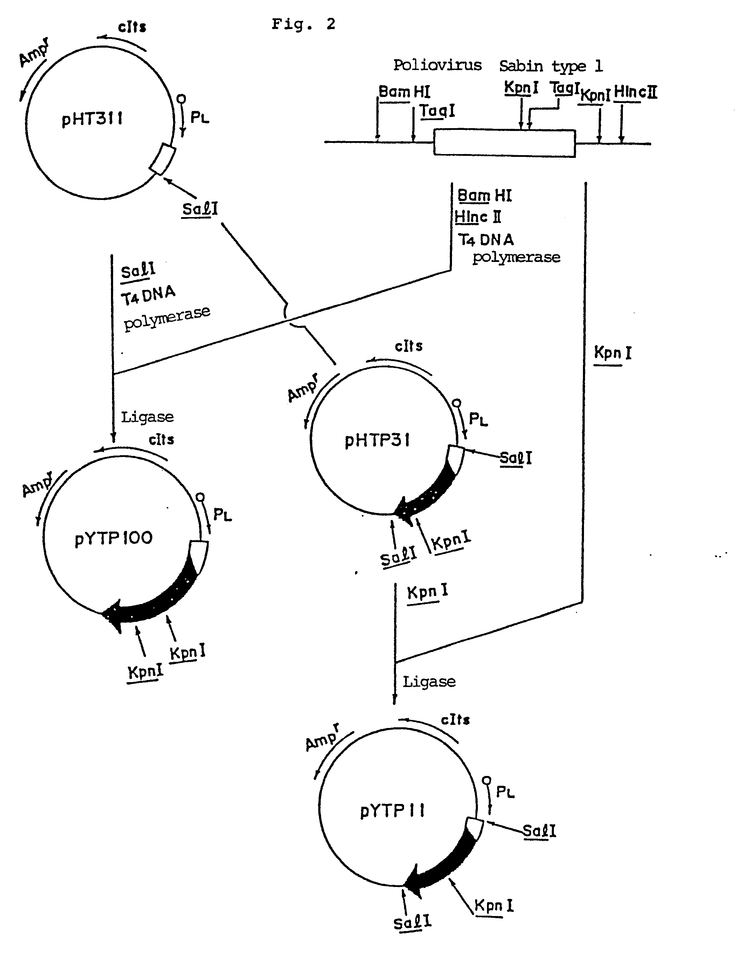

On the other hand, 5 µg of the pHTP31 plasmid was incubated with 10 U of Kpn I in 20 µl of a buffer (10 mM Tris-HCl pH 7.5, 7 mM $MgCl_2$, 7 mM β-mercaptoethanol). After ethanol precipitation, the precipitate was incubated with 1 U of alkaline phosphatase (bovine intestine) in 20 µl of a buffer (50 mM Tris-HCl pH 9.0, 1 mM $MgCl_2$, 0.1 mM $ZnCl_2$, 1 mM spermidine) at 37 °C for 30 minutes. After phenol treatment, ethanol precipitation was conducted. The precipitate was dissolved in 20 µl of 10 mM Tris-HCl pH 7.5, 1 mM EDTA solution. This solution contained 0.25 µg of DNA per 1 µl. 1 µg of this DNA and 0.3 µg of the DNA as described above were incubated with 2.8 U of $T_4$DNA ligase in 20 µl of a buffer (66 mM Tris-HCl pH 7.6, 6.6 mM $MgCl_2$, 10 mM DTT, 0.4 mM ATP). By use of this reaction mixture, Escherichia coli HB 101 strain was transformed, and the pYTP11 plasmid shown in Fig. 2 was isolated from among the ampicillin resistant transformants.

The cDNA of the poliovirus Sabin type 1 contained in this plasmid corresponds to the 1291 base pairs from the 2393th base to the 3683th base shown in the literature supra.

Example 3  Preparation of pYTP100

15 µg of the pBB2 plasmid was incubated with 30 U of Bam HI in 20 µl of a reaction medium (10 mM Tris-HCl pH 8.0, 7 mM $MgCl_2$, 100 mM NaCl, 2 mM β-mercaptoethanol) at 30 °C for 3 hours, followed by ethanol precipitation.  The precipitate was dissolved in 20 μl of a reaction medium (10 mM Tris-HCl pH 8.0, 7 mM $MgCl_2$, 60 mM NaCl, 7mM β-mercaptoethaol) and incubated with 30 U of Hinc II at 37 °C for 3 hours.  After ethanol precipitation, the precipitate was dissolved in 20 μl of a $T_4$DNA polymerase reaction solution and incubated with 2.5 U of $T_4$DNA polymerase at 37 °C for 15 minutes.  After deproteinization with phenol, the product was subjected to 1.2 % agarose gel electrophoresis, and a DNA fragment of about 1670 base pairs was recovered from the gel.  This was dissolved in 20 μl of 10 mM Tris-HCl pH 7.5, 1 mM EDTA solution.  This solution contained 0.07 μg of DNA per 1 μl.

0.5 μg of this DNA and 1 μg of the DNA fragment obtained from the pHT311 plasmid were incubated with 2.8 U of $T_4$DNA ligase in 20 μl of a $T_4$DNA reaction solution at 15 °C for 15 hours.  Escherichia coli HB 101 strain was transformed with this reaction product, and the pYTP100 plasmid as shown in Fig. 2 was isolated from among the ampicillin resistant transformants.

The cDNA of the poliovirus Sabin type 1 contained in this plasmid corresponds to the 1666 base pairs from the 2150th base to the 3915th base shown in the literature supra.

Example 4   Preparation of pYTP200

15 μg of the pVS(2)2503 plasmid [Toyoda, Haruka (Toyoda, H.), et al.; Journal of Molecular Biology (J. Mol. Biol.), 174, 561 (1984)] was incubated with 30 U of Kpn I in 20 μl of a buffer (6 mM Tris-HCl pH 7.5, 6 mM NaCl, 6 mM $MgCl_2$, 6 mM β-mercaptoethanol) at 37 °C for 3 hours.

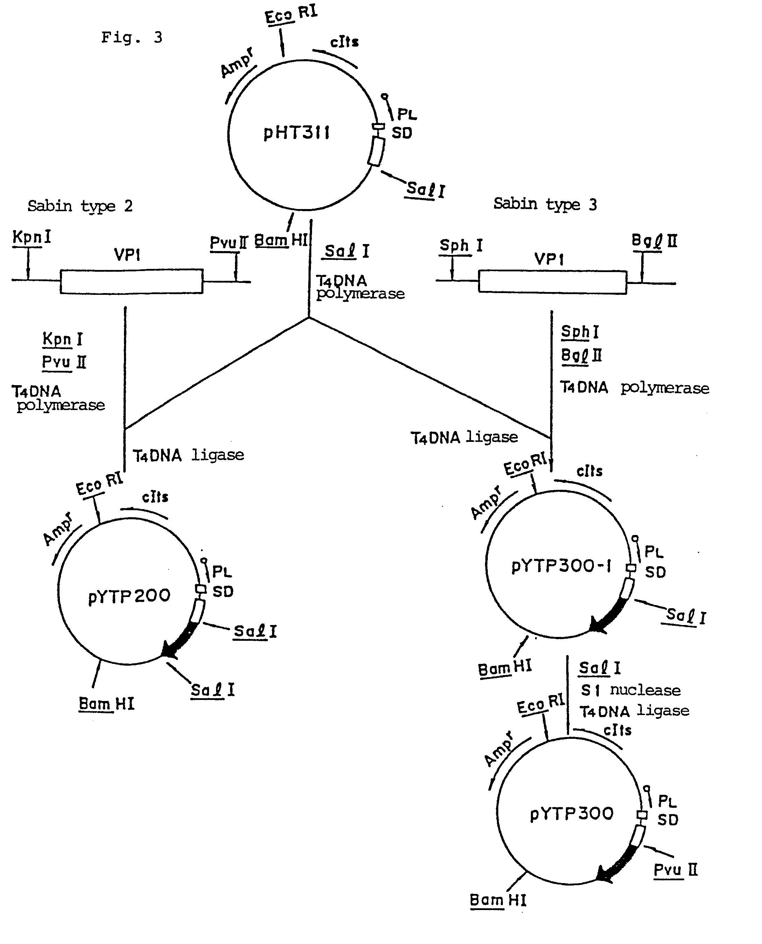

After ethanol precipitation, the precipitate was incubated with 32 U of Pvu II in 20 µl of a buffer (10 mM Tris-HCl pH 7.5, 7 mM $MgCl_2$, 60 mM NaCl, 7 mM β-mercaptoethanol) at 37 °C for 3 hours. This was subjected to 1.2 % agarose gel electrophoresis and a DNA fragment of about 1160 base pairs was recovered from the gel. This was incubated with 2.5 U of $T_4$DNA polymerase in 20 µl of a $T_4$DNA polymerase reaction solution (67 mM Tris-HCl pH 8.8, 6.7 mM $MgCl_2$, 10 mM β-mercaptoethanol, 6.7 mM EDTA, 16.6 mM $(NH_4)_2SO_4$, 330 µM dCTP, dATP, dGTP, dTTP) at 37 °C for 15 minutes. After phenol treatment, ethanol precipitation was conducted, and the precipitate was dissolved in 10 µl of 10 mM Tris-HCl pH 7.5, 1 mM EDTA solution. This solution contained 0.05 µg of DNA per 1 µl. 0.5 g of this DNA and 1 µg of the DNA fragment obtained from the pHT311 plasmid as described in Example 1 were incubated with 2.5 U of $T_4$DNA ligase in 20 µl of a buffer (66 mM Tris-HCl pH 7.6, 6.6 mM $MgCl_2$, 10 mM DTT, 1 mM ATP) at 15 °C for 5 hours. Escherichia coli HB 101 strain was transformed with this reaction product and the pYTP plasmid shown in Fig. 3 was isolated from among the ampicillin resistant transformants.

The cDNA of the poliovirus Sabin type 2 contained in this plasmid corresponds to the 1157 base pairs from the 2286th base to the 3442th base as shown in literature supra.

Example 5   Preparation of pYTP300

15 µg of the pVS(3)2603 plasmid [Toyoda, Haruka (Toyoda, H.), et al.; J. Mol. Biol., 174, 561 (1984)] was incubated with 30 U of SphI in 20 µl of a buffer (6 mM Tris-HCl pH 7.7, 125 mM NaCl, 6 mM $MgCl_2$, 7 mM β-mercaptoethanol, 0.01 % Triton X-100) at 37 °C for 3 hours. After ethanol precipitation, the precipitate was incu-

bated with 30U of <u>Bgl</u> II at 37 °C in 20 µl of a buffer (10 mM Tris-HCl pH 7.5, 7 mM $MgCl_2$, 60 mM NaCl, 7mM β-mercaptoethanol) at 37 °C for 3 hours, and then subjected to 1.2 % agarose gel electrophoresis. A DNA fragment of about 1040 base pairs was recovered from the gel. This was incubated with 2.5 U of $T_4$DNA polymerase in a buffer (67 mM Tris-HCl pH 8.8, 6.7 mM $MgCl_2$, 10 mM β-mercaptoethanol, 6.7 mM EDTA, 16.6 mM $(NH_4)_2SO_4$, 330 µM dCTP, dATP, dGTP, dTTP) at 37 °C for 15 minutes. After phenol treatment, ethanol precipitation was conducted, and the precipitate was dissolved in 20 µl of 10 mM Tris-HCl pH 75, 1 mM EDTA solution. This solution contained 0.05 µg of DNA per 1 µl. 0.5 g of this DNA and 1 µg of the DNA fragment obtained from the pHT311 plasmid as described in Example 1 were incubated with 2.5 U of $T_4$DNA ligase in 20 µl of a buffer (66 mM Tris-HCl pH 7.6, 6.6 mM $MgCl_2$, 10 mM DTT, 1 mM ATP) at 15 °C for 15 hours. <u>Escherichia coli</u> HB 101 strain was transformed with this reaction product and the pYTP 300-1 plasmid shown in Fig. 3 was isolated from among the ampicillin resistant transformants.

Next, 5 µg of the pYTP 300-1 plasmid was incubated with 10 U of <u>Sal</u> I in 20 µl of a buffer (6 mM Tris-HCl pH 7.9, 150 mM NaCl, 6 mM $MgCl_2$, 6 mM β-mercaptoethanol) at 37 °C for 3 hours. After ethanol precipitation, the precipitate was incubated with 50U of S1 nuclease in 20 µl of a buffer (50 mM $CH_3COONa$, 200 mM NaCl, 1 mM $ZnSO_4$, 0.5 % glycerol) at 37 °C for 30 minutes. After phenol treatment, ethanol precipitation was conducted and the precipitate was incubated with 2.5 U of $T_4$DNA ligase in 20 µl of a buffer (66 mM Tris-HCl pH 7.6, 6.6 mM $MgCl_2$, 10 mM DTT, 1 mM ATP) at 15 °C for 15 hours.

<u>Escherichia coli</u> HB 101 strain was transformed with this reaction product and the pYTP 300 plasmid shown in Fig. 3 was isolated from among the ampicillin resistant transformants.

The cDNA of the poliovirus Sabin type 3 contained in this plasmid corresponds to the 1041 base pairs from the 2416th base to the 3456th base as shown in literature supra.

Example 6   Preparation of pYTP102

5 µg of the pYTU3 plasmid was incubated with 5 U of Sal I in 20 µl of a buffer (10 mM Tris-HCl pH 7.5, 7 mM $MgCl_2$, 150 mM NaCl, 0.2 mM EDTA, 7 mM β-mercaptoethanol) at 37 °C for 3 hours.  After ethanol precipitation, the precipitate was dissolved in 20 µl of 10 mM Tris-HCl pH 7.5, 1 mM EDTA solution.  On the other hand, of the cDNA of the poliovirus Sabin type 1, 15 µg of the pHTP31 plasmid containing the DNA region coding for the VP1 protein was incubated with 20 U of Sal I in 20 µl of a buffer (10 mM Tris-HCl pH 7.5, 7 mM $MgCl_2$, 150 mM NaCl, 0.2 mM EDTA, 7 mM β-mercaptoethanol) at 37 °C for 3 hours.  After the incubation, 1.2 % agrose electrophoresis was carried out, and a DNA fragment of 713 base pairs was extracted according to the conventional method.  After ethanol precipitation, the precipitate was dissolved in 20 µl of 10 mM Tris-HCl pH 7.5, 1 mM EDTA solution.  This DNA fragment corresponds to the 713 base pairs from the 2373th base to the 3085th base shown in the literature [Nomoto, Akio (Nomoto, A.), et al.; Proceeding National Academic Science USA (Proc. Natl. Acad. Sci. USA), 79, 5793 (1982)].

0.5 µg of this DNA fragment and 0.2 µg of the DNA obtained by cleavage of the pYTU3 plasmid with Sal I were incubated with 2.5 U of $T_4$DNA ligase in 20 µl of a buffer (66 mM Tris-HCl pH 7.6, 6.6 mM $MgCl_2$, 10 mM DTT, 1 mM ATP) at 15 °C for 15 hours.

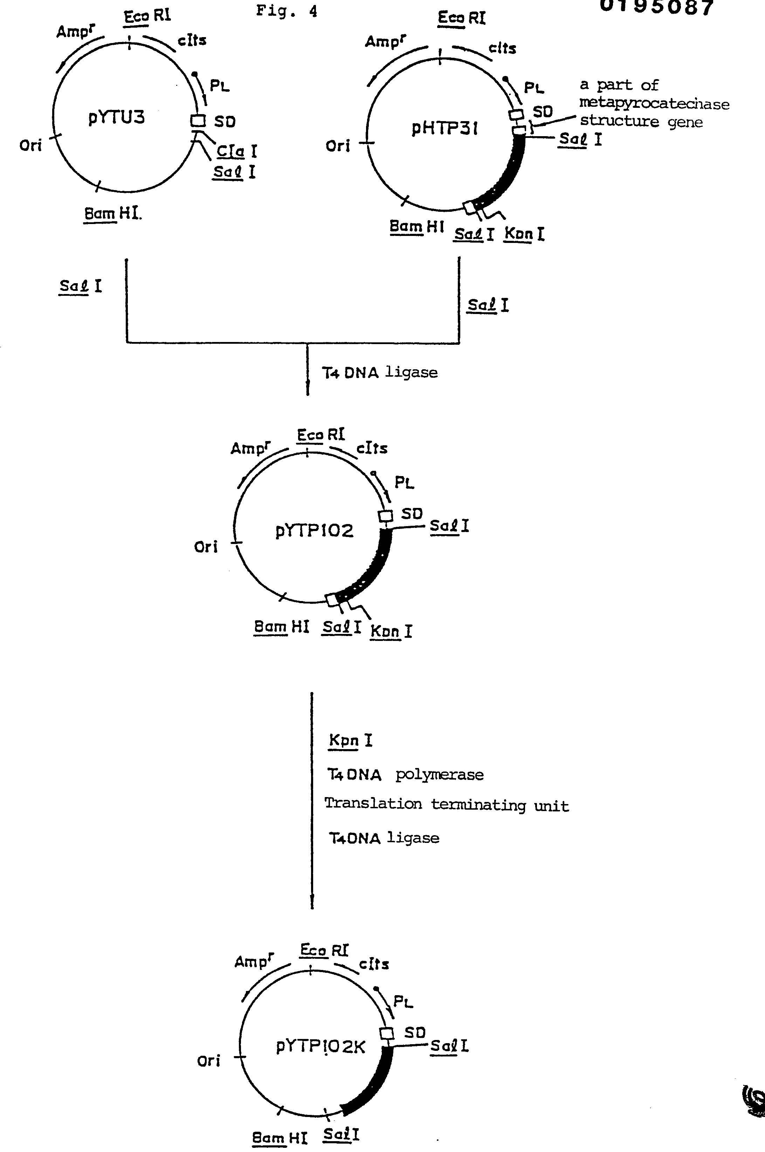

With the use of this reaction mixture, _Escherichia_ _coli_ HB101 strain was transformed, and the pYTP 102 plasmid shown in Fig. 4 was isolated from among the ampicillin resistant microorganisms. Next, following a conventional method, _Escherichia_ _coli_ 600 strain was transformed with the pYTP 102 plasmid, and the _Escherichia_ _coli_ 600/pYTP 102 was isolated.

Example 7   Preparation of pYTP102K

1 µg of the pYTP102 plasmid was incubated with 5 U of _Kpn_ I in 20 µl of a buffer (10 mM Tris-HCl pH 7.5, 7 mM $MgCl_2$, 7 mM β-mercaptoethanol) at 37 $^\circ$C for 2 hours. After ethanol precipitation, the precipitate was incubated with 2.8 U of $T_4$DNA polymerase in 20 µl of a buffer (67 mM Tris-HCl pH 8.8, 6.7 mM $MgCl_2$, 10 mM β-mercaptoethanol, 6.7 mM EDTA, 16.6 mM $(NH_4)_2SO_4$, 330 µM dCTP, dATP, dGTP, dTTP) at 37 $^\circ$C for 15 minutes. After phenol treatment, ethanol precipitation was conducted, and the precipitate and 0.5 µg of a commercially available Universal Translation Terminator Oligonucleotide (sold by Pharmacia P-L Biochemical) phosphorylated at 5'-end by treatment with $T_4$ polynucleotide kinase were incubated with 2.5 U of $T_4$DNA ligase in 20 µl of a buffer (66 mM Tris-HCl pH 7.6, 6.6 mM $MgCl_2$, 10 mM DTT, 1 mM ATP) at 15 $^\circ$C for 15 hours. With the use of this reaction mixture, _Escherichia_ _coli_ HB101 strain was transformed, and the pYTP 102K plasmid shown in Fig. 4 was isolated from among the ampicillin resistant microorganisms. Next, following a conventional method, _Escherichia_ _coli_ C600 strain was transformed with the pYTP 102K plasmid, and the _Escheri_-_chia_ _coli_ 600/pYTP 102K was isolated.

Example 8   Preparation of pYTP102P

10 µg of the pYTP plasmid was incubated with 30 U of Pst I in 20 µl of a buffer (10 mM Tris-HCl pH 7.5, 7 mM

MgCl$_2$, 50 mM (NH$_4$)$_2$SO$_4$) at 37 $^\circ$C for 3 hours. After ethanol precipitation, the precipitate was incubated with 2.8 U of T$_4$DNA polymerase in 20 µl of a buffer (67 mM Tris-HCl pH 8.8, 6.7 mM MgCl$_2$, 10 mM β-mercaptoethanol, 6.7 mM EDTA, 16.6 mM (NH$_4$)$_2$SO$_4$, 330 µM dCTP, dATP, dGTP, dTTP) at 37 $^\circ$C for 15 minutes. After phenol treatment, ethanol precipitation was conducted, and the precipitate and 0.5 µg of a commercially available Universal Translation Terminator Oligonucleotide (sold by Pharmacia P-L Biochemical) phosphorylated at the 5'-end by treatment with T$_4$polynucleotide kinase were incubated with 2.5 U of T$_4$DNA ligase in 20 µl of a buffer (66 mM Tris-HCl pH 7.6, 6.6 mM MgCl$_2$, 10 mM DTT, 1 mM ATP) at 15 $^\circ$C for 15 hours. After ethanol precipitation, the precipitate was incubated with 30 U of Sal I in 20 µl of a buffer (10 mM Tris-HCl pH 7.5, 7 mM MgCl$_2$, 150 mM NaCl, 0.2 mM EDTA, 7 mM β-mercaptoethanol) at 37 $^\circ$C for 3 hours, followed by 1.2 % agarose gel electrophoresis. A DNA fragment of about 1050 base pairs was recovered following a conventional method.

On the other hand, 5 µg of the pYTU3 plasmid was incubated with 16 U of Nru I in 20 µl of a buffer (6 mM Tris-HCl pH 7.4, 6 mM MgCl$_2$, 100 mM NaCl, 5 mM β-mercaptoethanol) at 37 $^\circ$C for 3 hours. After ethanol precipitation, the precipitate was incubated with 20 U of Sal I in 20 µl of a buffer (10 mM Tris-HCl pH 7.5, 7 mM MgCl$_2$, 150 mM NaCl, 0.2 mM EDTA, 9 mM β-mercaptoethanol) at 37 $^\circ$C for 3 hours. By carrying out 1.2 % agarose gel electrophoresis, a DNA fragment of about 4000 base pairs was recovered.

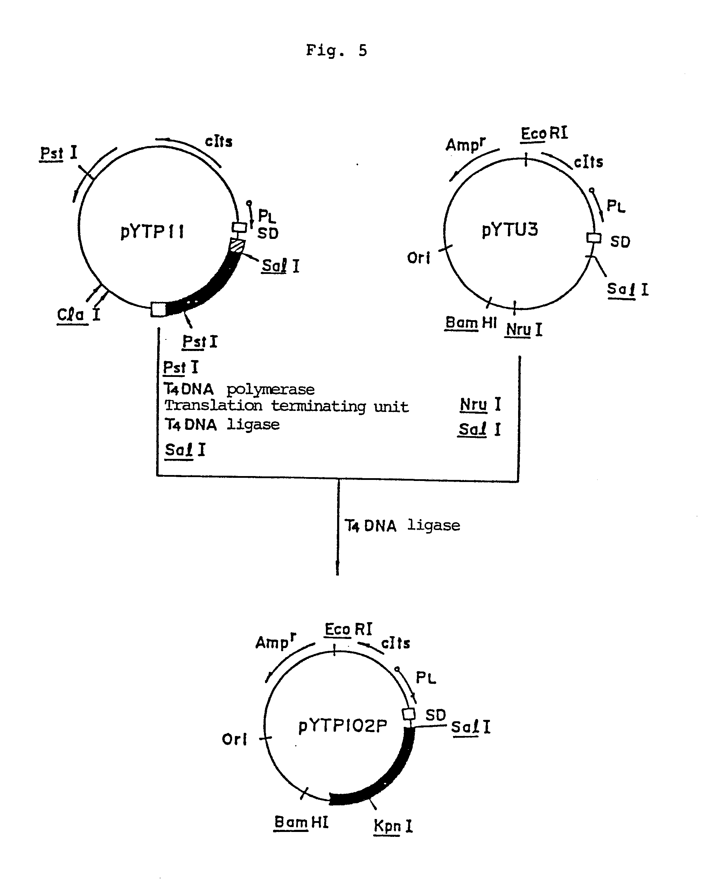

0.5 µg of the above DNR fragment of about 1050 base pairs and 1.0 µg of the DNA fragment of about 4000 base pairs were incubated with 2.5 U of T$_4$DNA ligase in 20 µl of a buffer (66 mM Tris-HCl pH 7.6, 6.6 mM MgCl$_2$, 10 mM DTT, 1 mM ATP) at 15 °C for 15 hours. With the use of this reaction mixture, Escherichia coli HB101 strain was transformed, and the pYTP 102P plasmid shown in Fig. 5 was isolated from among the ampicillin resistant microorganisms. Next, following a conventional method, Escherichia coli C600 strain was transformed with the pYTP 102P plasmid, and the Escherichia coli 600/pYTP102P was isolated.

Example 9  Preparation of pYTP201N

10 μg of the pYTP200 plasmid was incubated with 30 U of Sal I in 20 μl of a buffer (10 mM Tris-HCl pH 7.5, 7 mM MgCl$_2$, 150 mM NaCl, 0.2 mM EDTA, 7 mM β-mercaptoethanol) at 37 °C for 3 hours. By carrying out 1.2 % agarose gel electrophoresis, a DNA fragment of about 1160 base pairs was recovered.

On the other hand, 5 μg of the pYTU3 plasmid was incubated with 20 U of Sal I in 20 μl of a buffer (10 mM Tris-HCl pH 7.5, 7 mM MgCl$_2$, 150 mM NaCl, 0.2 mM EDTA, 7 mM β-mercaptoethanol) at 37 °C for 3 hours. By carrying out 1.2 % agarose gel electrophoresis, a DNA fragment of about 4500 base pairs was recovered.

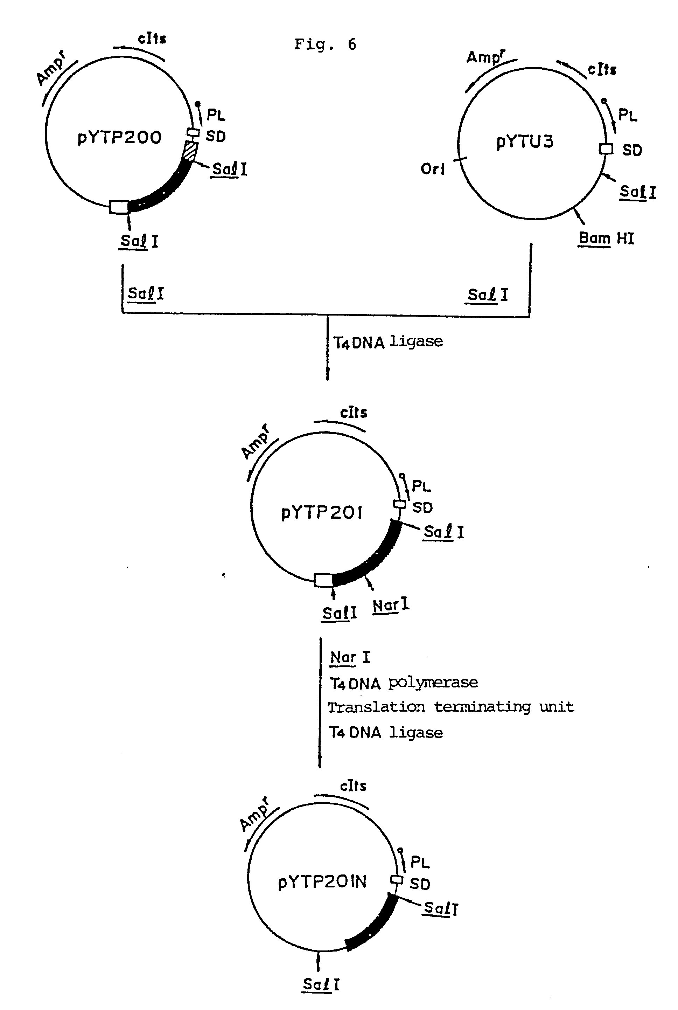

0.5 μg of the above DNR fragment of about 1160 base pairs and 1.0 μg of the DNA fragment of about 4500 base pairs were incubated with 2.5 U of T$_4$DNA ligase in 20 μl of a buffer (66 mM Tris-HCl pH 7.6, 6.6 mM MgCl$_2$, 10 mM DTT, 1 mM ATP) at 15 °C for 15 hours. With the use of this reaction mixture, Escherichia coli HB101 strain was transformed, and the pYTP 201 plasmid shown in Fig. 6 was isolated from among the ampicillin resistant microorganisms.

2 μg of the pYTP201 plasmid was incubated with 10 U of Nar I in 20 μl of a buffer (6 mM Tris-HCl pH 7.4, 6 mM

$MgCl_2$, 50 mM NaCl, 6 mM β-mercaptoethanol) at 37 $^O$C for 3 hours.  After ethanol precipitation, the precipitate was incubated with 2.8 U of $T_4$DNA polymerase in 20 μl of a buffer (67 mM Tris-HCl pH 8.8, 6.7 mM $MgCl_2$, 10 mM β-mercaptoethanol, 6.7 mM EDTA, 16.6 mM $(NH_4)_2SO_4$, 330 μM dCTP, dATP, dGTP, dTTP) at 37 $^O$C for 15 minutes.  After phenol treatment, ethanol precipitation was conducted, and the precipitate and 0.5 μg of a commercially available Universal Translation Terminator Oligonucleotide (sold by Pharmacia P-L Biochemical) phosphorylated at 5'-end by treatment with $T_4$polynucleotide kinase were incubated with 2.5 U of $T_4$DNA ligase in 20 μl of a buffer (66 mM Tris-HCl pH 7.6, 6.6 mM $MgCl_2$, 10 mM DTT, 1 mM ATP) at 15 $^O$C for 15 hours.  With the use of this reaction mixture, Escherichia coli HB101 strain was transformed, and the pYTP 201N plasmid shown in Fig. 6 was isolated from among the ampicillin resistant microorganisms.  Next, following a conventional method, Escherichia coli C600 strain was transformed with the pYTP 201N plasmid, and the Escherichia coli 600/pYTP 201N was isolated.

Example 10  Preparation of pYT301K

10 μg of the pYTP300 plasmid was incubated with 20 U of Pst I in 20 μl of a buffer (10 mM Tris-HCl pH 7.5, 7 mM $MgCl_2$, 50 mM $(NH_4)_2SO_4$) at 37 $^O$C for 3 hours.  After ethanol precipitation, the precipitate was incubated with 2.8 U of $T_4$DNA polymerase in 20 μl of a buffer (67 mM Tris-HCl pH 8.8, 6.7 mM $MgCl_2$, 10 mM β-mercaptoethanol, 6.7 mM EDTA, 16.6 mM $(NH_4)_2SO_4$, 330 μM dCTP, dATP, dGTP, dTTP) at 37 $^O$C for 15 minutes.  After phenol treatment, ethanol precipitation was conducted, and the precipitate was incubated with 30 U of Bam HI  in 20 μl of a buffer (10 mM Tris-HCl pH 8.0, 7 mM $MgCl_2$, 100 mM NaCl, 2 mM β-mercaptoethanol) at 30 $^O$C for 3 hours.  By carrying out 1.2 % agarose gel electrophoresis, a DNA fragment of about 1500 base pairs was recovered.

On the other hand, 5 µg of the pYTU3 plasmid was incubated with 20 U of <u>Sal</u> I in 20 µl of a buffer (10 mM Tris-HCl pH 7.5, 7 mM $MgCl_2$, 150 mM NaCl, 0.2 mM EDTA, 7 mM β-mercaptoethanol) at 37 °C for 3 hours. After ethanol precipitation, the precipitate was incubated with 10 U of S1 nuclease in 100 µl of a buffer (50 mM $CH_3$-COONa, 200 mM NaCl, 1 mM $ZnSO_4$, 0.5 % glycerol) at 37 °C for 15 minutes. After phenol treatment, ethanol precipitation was conducted and the precipitate was incubated with 30 U of <u>Bam</u> HI in 20 µl of a buffer (10 mM Tris-HCl pH 8.0, 7 mM $MgCl_2$, 100 mM NaCl, 2 mM β-mercaptoethanol) at 30 °C for 3 hours. By carrying out 1.2 % agarose gel electrophoresis, a DNA fragment of about 3800 base pairs was recovered.

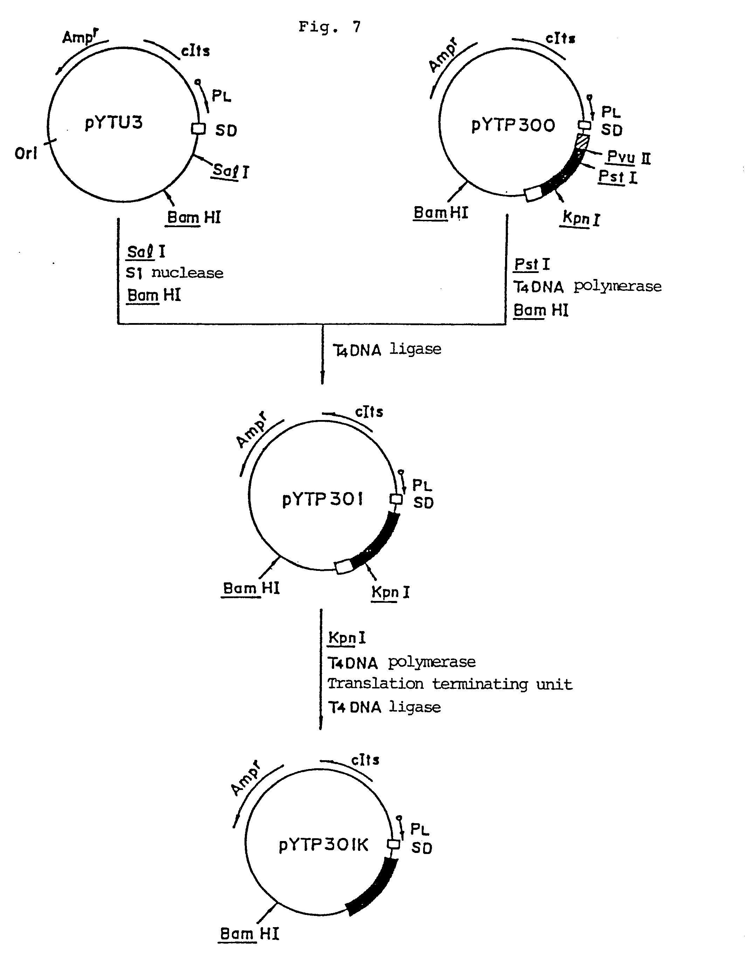

0.5 µg of the above DNR fragment of about 1500 base pairs and 1.0 µg of the DNA fragment of about 3800 base pairs were incubated with 2.5 U of $T_4$DNA ligase in 20 µl of a buffer (66 mM Tris-HCl pH 7.6, 6.6 mM $MgCl_2$, 10 mM DTT, 1 mM ATP) at 15 °C for 15 hours. With the use of this reaction mixture, <u>Escherichia</u> <u>coli</u> HB101 strain was transformed, and the pYTP 301 plasmid shown in Fig. 7 was isolated from among the ampicillin resistant microorganisms.

2 µg of the pYTP201 plasmid was incubated with 5 U of <u>Kpn</u> I in 20 µl of a buffer (10 mM Tris-HCl pH 7.5, 7 mM $MgCl_2$, 7 mM β-mercaptoethanol) at 37 °C for 3 hours. After ethanol precipitation, the precipitate was incubated with 2.8 U of $T_4$DNA polymerase in 20 µl of a buffer (67 mM Tris-HCl pH 8.8, 6.7 mM $MgCl_2$, 10 mM β-mercapto-ethanol, 6.7 mM EDTA, 16.6 mM $(NH_4)_2SO_4$, 330 µM dCTP, dATP, dGTP, dTTP) at 37 °C for 15 minutes. After phenol treatment, ethanol precipitation was conducted, and the precipitate and 0.5 µg of a commercially available Universal Translation Terminator Oligonucleotide (sold by Pharmacia P-L Biochemical) phosphorylated at 5'-end by treatment with $T_4$polynucleotide kinase were incubated with 2.5 U of $T_4$DNA ligase in 20 µl of a buffer (66 mM Tris-HCl pH 7.6, 6.6 mM $MgCl_2$, 10 mM DTT, 1 mM ATP) at 15 °C for 15 hours. With the use of this reaction mixture, Escherichia coli HB101 strain was transformed, and the pYTP 301K plasmid shown in Fig. 7 was isolated from among the ampicillin resistant microorganisms. Next, following a conventional method, Escherichia coli C600 strain was transformed with the pYTP 301K plasmid, and the Escherichia coli 600/pYTP 301K was isolated.

Example 11  Preparation of VP1 fused protein

Escherichia coli C600 strain retaining the pHTP31 plasmid was subjected to shaking cultivation in a test tube containing 5 ml of L medium (ampicillin conc. 25 µg/ml) at 28 °C for an overnight. When $OD_{650}$ of the culture broth became 0.8, it was immediately transferred into a thermostat bath at 42 °C, wherein shaking cultivation was carried out. Then, 1 ml of the culture broth was transferred into an Eppendorf tube and centrifuged. The supernatant was removed and 1 ml of a solution of 10 mM Tris-HCl pH 7.5, 1 mM DTT, 23 µM p-methylsulfonic acid fluoride (hereinafter called "PMSF") was added to the precipitated microorganism cells, and the cells were crushed by sonication on ice. The crushed solution was separated and, after removal of the supernatant, 20 µl of 6 M urea solution was added, followed by thorough stirring. After left to stand on ice-bath for 30 minutes, the mixture was subjected to centrifugation.

The supernatant was added with an equal amount of a sample buffer for electrophoresis (finally 10 mM Tris-HCl

- 64 -

0195087

pH 8.0, 1 mM EDTA, 10 % sucrose, 40 mM DTT, 1 % SDS) and subjected to SDS-polyacrylamide gel electrophoresis (hereinafter called "SDS-PAGE"). The same operations were conducted for the Escherichia coli strain C600 strain retaining no pHTP31 plasmid. Next, Coomassie blue staining and enzyme antibody staining were performed, with the result that a band attributable to the VP1 fused protein with a molecular weight of about 29,000 was detected for the C600/pHTP31 strain. For the C600 strain having no plasmid, this band was not detected. Also, by performing enzyme antibody staining, this protein was confirmed to react with the antibody against Sabin type 1 virus.

The content of the VP1 fused protein was measured by a densitometer to be 24.8 %. This corresponds to 19.4 mg as calculated on the expressed amount per 1 liter of the culture broth.

Example 12    Preparation of VP1 fused protein

After 1 ml of the culture broth of the transformed strain Escherichia coli C600/pHTP31 cultured under the same conditions as Example 11 was centrifuged, microorganisms cells were treated with lysozyme chloride (egg white) and Triton X100 ("Biochemical Experimental Course Vol. 5, Enzyme Study Method (part 1)", ed. by Society of Biochemistry of Japan, Tokyo Kagaku Dojin) to be crushed. The precipitate after centrifugation was added with 6M urea solution, stirred well and then left to stand on ice-bath for 30 minutes, followed by centrifugation. An equal amount of sample buffer for electrophoresis was added to the supernatant, and the mixture was subjected to SDS-PAGE. Then, enzyme antibody staining was performed. The content of the VP1 fused protein was measured by densitometry to be 14.5 %. This corresponds to 10.2 mg as calculated on the expressed amount per 1 liter of the culture broth.

Example 13   Preparation of VP1 fused protein

For the transformed strain Escherichia coli C600 retaining the pYTP11 plasmid or the pYTP100 plasmid, according to the same method as Example 11, the VP1 fused protein was extracted from the precipitate after sonication with 6 M urea solution and subjected to SDS-PAGE.

In C600/pYTP11, some proteins were detected with a protein of about 46,000 molecular weight being the longest. On the other hand, in C600/pYTP100, some proteins were detected with a protein of about 69,000 molecular weight being the longest. Further, enzyme antibody staining was performed to confirm that these proteins reacted with the antibody against Sabin type 1 virus.

The contents of the VP1 fused proteins were measured by densitometry to be 28.9 % and 15.5 %, respectively. These correspond to 22.6 mg and 12.1 mg, respectively, as calculated on the expressed amounts per 1 liter of the culture broth.

Example 14   Preparation of VP1 fused protein

For the transformed strain Escherichia coli C600 retaining the pYTP200 plasmid or the pYTP300 plasmid, according to the same method as Example 11, the VP1 fused protein was extracted from the precipitate after sonication with 6 M urea solution and subjected to SDS-PAGE.

In C600/pYTP200, a protein of about 52,000 molecular weight was detected. On the other hand, in C600/pYTP300, some proteins were detected with a protein of about 40,000 molecular weight being the longest. Further, enzyme antibody staining was performed to confirm that these proteins reacted with the antibody against Sabin type 1 virus.

The contents of the VP1 fused proteins were measured by densitometry to be 14.9 % and 5.0 %, respectively. These correspond to 11.7 mg and 3.9 mg, respectively, as calculated on the expressed amounts per 1 liter of the culture broth.

Example 15   Preparation of VP1 fused protein

Shaking cultivation of a transformant _Escherichia coli_ C600 strain retaining the pHTP31 strain was carried out in a 2-liter culture flask containing one liter of L medium (ampicillin conc. 25 µg/ml) at 30 $^{O}$C, and the culture broth, on reaching an $OD_{650}$ value of 0.8, was immediately transferred into a thermostat bath at 42 $^{O}$C, wherein shaking cultivation was continued for 3 hours. After a predetermined period of time, the microorganism cells were collected by centrifuge and subjected to sonication with addition of 100 ml of 10 mM Tris-HCl pH 7.5, 1 mM DTT, 23 µM PMSF solution to crush the microorganism cells.

After centrifugation, the precipitate was well stirred with addition of 10 ml of 6 M urea solution, and left to stand on ice-bath for 30 minutes. Further, after centrifugation, the supernatant was dialyzed against 50-fold volume of 10 mM Tris-HCl pH 7.5, 1 mM EDTA, 1 mM DTT solution, which was repeated for 4 times, followed by SDS-PAGE.

The content of the VP1 fused protein was measured by densitometry to be 28.1 %. This corresponds to 21.6 mg as calculated on the expressed amount per 1 liter of the culture broth.

Example 16   Preparation of pSLMPK1

14 µg of the pBB2 plasmid was incubated with 100 U of Bam HI in 120 µl of a reaction medium (10 mM Tris-HCl pH 8.0, 7 mM $MgCl_2$, 0.1 M NaCl, 2 mM β-mercaptoethanol) at 30 °C for 5 hours.  The reaction mixture was subjected to 0.7 % agarose gel electrophoresis for separation, and a DNA fragment of 2500 base pairs was extracted according to a conventional means and dissolved in 20 µl of water.  To 16 µl of this solution, 5 µl of a buffer (100 mM Tris-HCl pH 8.4, 1 M NaCl, 60 mM $MgCl_2$, 60 mM β-mercaptoethanol), 27 µl of water and 2 µl (20 U) of Taq I, and incubation was conducted at 65 °C for 3.5 hours.  The reaction mixture was subjected to 0.7 % agarose gel electrophoresis for separation, and a DNA fragment of about 700 base pairs was extracted and dissolved in 8 µl of water.

After 5.5 µg of the pSLMK1 plasmid was incubated with 20 U of Sal I in 100 µl of a reaction medium (6 mM Tris-HCl pH 7.9, 150 mM NaCl, 6 mM $MgCl_2$, 6 mM β-mercaptoethanol) at 37 °C for 3 hours, the reaction mixture was subjected to 0.7 % agarose gel electrophoresis for separation, a DNA fragment of about 3200 base pairs was extracted and dissolved in 50 µl of water.  To 10 µl of this solution were added 2.5 µl of a buffer (500 mM Tris-HCl pH 7.2, 100 mM $MgCl_2$, 1 mM DTT), 1 µl of 2 mM dNTP, 10.5 µl of water and 1 µl (5.2 U) of DNA polymerase I Klenow fragment, followed by incubation at 22 °C for 30 minutes. The reaction mixture was heated at 70 °C for 5 minutes. After left to cool, 25 µl of the solution was incubated with addition of 10 µl (0.005 OD) of the Cla I linker [d(CATCCATG)] phosphorylated at the 5'-end, 1 µl of a buffer (660 mM Tris-HCl pH 7.6, 66 mM $MgCl_2$, 50 mM DTT,

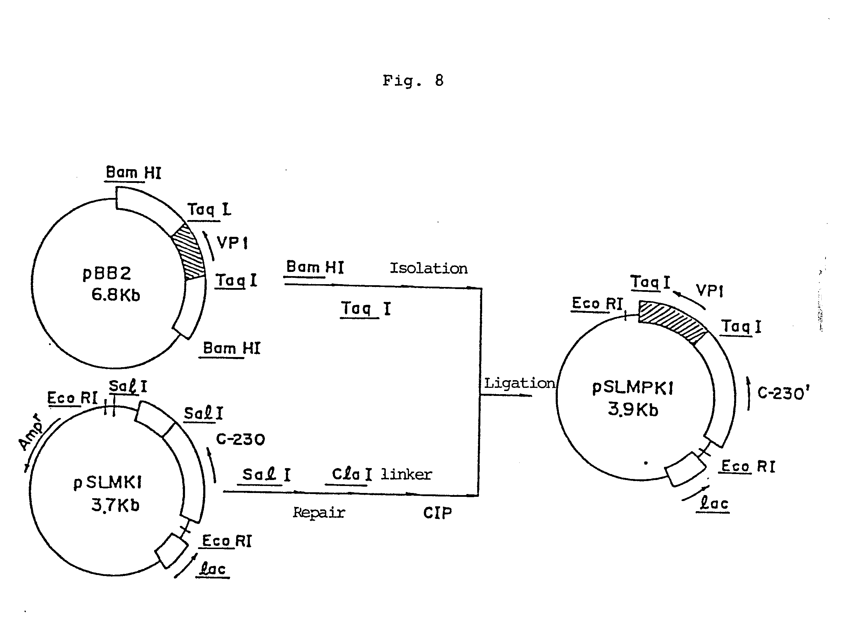

10 mM ATP) and 1 µl (2.8 U) of $T_4$DNA ligase at 22 $^{O}$C for 6 hours.  After the reaction, phenol extraction and ethanol precipitation were conducted, and then the precipitate was dissolved in 90 µl of water.  To this solution were added 10 µl of a buffer (60 mM Tris-HCl pH 7.9, 60 mM MgCl$_2$, 500 mM NaCl) and 4 µl (20 U) of Cla I, and incubation was carried out at 37 $^{O}$C for 4 hours. After the reaction, the reaction was stopped with addition of 4 µl of 0.25 M EDTA and, after phenol extraction and ethanol precipitation, the precipitate was subjected to 0.7 % agarose gel electrophoresis for separation, and a DNA fragment of 2500 base pairs was extracted and dissolved in 20 µl of water.  To 18 µl of this DNA fragment solution were added 5 µl of a buffer (500 mM Tris-HCl pH 9.0, 10 mM MgCl$_2$, 1 mM ZnCl$_2$, 10 mM spermidine) and 1 U of alkaline phosphatase (bovine intestine), and incubation was carried out at 37 $^{O}$C for 30 minutes. After the incubation, the reaction was stopped with addition of 1 µl of 0.25 M EDTA and, after phenol extraction and ethanol precipitation, the precipitate was dissolved in 2 µl of TE buffer (pH 8.0).  To 2 µl of this solution were added 4 µl of the Taq I fragment obtained from the pBB2 plasmid as described above, 1 µl of a buffer (660 mM Tris-HCl pH 7.6, 66 mM MgCl$_2$, 50 mM DTT, 10 mM ATP), 2 µl of water and 1 µl (2.8 U) of $T_4$DNA ligase, and incubation was carried out at 12 $^{O}$C for 12 $^{O}$C.  After the incubation, Escherichia coli RR1 strain was transformed with the use of this solution, the colony was screened with ampicillin resistance as the marker, and the pSLMPK1 plasmid as shown in Fig. 8 was isolated according to a conventional means.

Example 17   Preparation of VP1 fused protein

Shaking cultivation of Escherichia coli C600 strain retaining the pSLMPK1 plasmid was carried out in a test tube containing 5 ml of L medium (ampicillin conc. 25

μg/ml) at 37 °C, and the culture broth, on reaching an $OD_{650}$ value of 0.8, was immediately transferred in an amount of 1 ml into an Eppendorf tube and centrifuged. Subsequently, the same operations as Example 11 were performed.

By SDS-PAGE, a band attributable to the VP1 fused protein with a molecular weight of about 60,000 dalton was detected. Also, it was confirmed by enzyme antibody staining that said protein reacted with the antibody against Sabin type 1 virus.

Next, the content of the VP1 fused protein was measured by densitometry to be 24.1 %. This corresponds to 18.6 mg as calculated on the expressed amount per 1 liter of the culture broth.

Example 18  Preparation of VP1 non-fused protein

Shaking cultivation of Escherichia coli C600 strain retaining the pYTP 102P plasmid was carried out in a test tube containing 1 ml of L medium (ampicillin conc. 25 μg/ml) at 28 °C for an overnight. When the $OD_{650}$ value of the culture broth became 0.8, it was immediately transferred into a thermostat bath at 42 °C and shaking cultivation was conducted for 3 hours. After centrifugation, the supernatant was removed, and the precipited microorganism cells were crushed by sonication at 2 °C with addition of 100 ml of 10 mM Tris-HCl pH 7.5, 1 mM EDTA solution. The crushed solution was separated and, after removal of the supernatant, stirred well with addition of 10 ml of 6 M urea solution. After left to stand on icebath for 30 minutes, the mixture was centrifuged. Further, the supernatant was dialyzed against 100-fold volume of 10 mM Tris-HCl pH 7.5, 1 mM EDTA solution for 3 times.

To a portion of the supernatant was added an equal amount of a sample buffer for electrophoresis (finally 10 mM Tris-HCl pH 8.0, 1 mM EDTA, 10 % sucrose, 40 mM DTT, 1 % SDS), and the mixture was subjected to SDS-polyacrylamide gel electrophoresis. Then, Coomassie blue staining was performed and a band attributable to the VP1 protein with a molecular weight of about 39,000 dalton was detected.

The content of the VP1 fused protein was measured by densitometry to be 20.3 %. This corresponds to 9.6 mg as calculated on the expressed amount per 1 liter of the culture broth. Also, it was confirmed by enzyme antibody staining that this protein reacted with the antibody against Sabin type 1 virus.

The same operations were conducted also for Escherichia coli C600/pYTP102K, C600/pYTP201N and C600/pYTP301K, and VP1 non-fused proteins with molecular weights of 25,500, 29,000 and 25,500 were obtained, respectively. These were confirmed by enzyme antibody staining to be reactive with antibodies against Sabin type 1 virus, Sabin type 2 virus and Sabin type 3 virus, respectively. The contents of the VP1 proteins in the solutions extracted of the precipitates after sonication with 6 M urea solution and the expressed amounts per 1 liter of the culture broth are shown in the following Table.

Table

| | Content (%) | Expressed amount per liter of broth (mg) |
|---|---|---|
| Escherichia coli C600/pYTP102K | 3.5 | 1.7 |
| Escherichia coli C600/pYTP201N | 22.9 | 10.8 |
| Escherichia coli C600/pYTP301K | 4.0 | 1.9 |

[Utilizability in industry]

According to the present invention, the protein having the antigenic determinant of the polyovirus capsid protein VP1 can be supplied in a large amount in the form of fused protein or non-fused protein. The active protein thus obtained can be used as the reagent for detection of polyovirus antibody, for example, for radioimmunoassay. Also, by administering parenterally to mammals, antibody against the poliovirus capsid VP1 protein can be produced and therefore it is useful for polyovirus vaccine. Further, the side effect of live vaccine can be reduced when it is used for preimmunization for administration of live vaccine.

0195087

Format 2

NOTICE OF REFUSAL TO RECEIVE DEPOSITION

Notice No.: 60 FERM Doc. No. 270 Date of notice: March 9, 1985

Esq. Foundation Sagami Chemical Research Center
Kiyosi Kondo, Vice chairman, and other 5 (as
per attached sheet) President of Institute of Fermentation Research, Agency of
Industrial Science & Technology
Jiro Oyama (sealed)

We hereby notice that the microorganism applied by you to be deposited cannot be received for the reason shown below.

Remarks

1. Date of application:
   February 27, 1985

2. Representation for discrimination of microorganism:
   Escherichia coli C600/pYTP102K

3. Reason for refusal:
   Because of outside of the range of microorganisms which can be received.

0195087

Attached sheet

Names of depositors:

1. Foundation Sagami Chemical Research Center
    Vice chairman: Kiyosi Kondo

2. Central Glass Co., Ltd.
    President: Mitsuyoshi Ito

3. Hodogaya Chemical Co., Ltd.
    President: Kenichi Fujioka

4. Nippon Soda Co., Ltd.
    President: Takeo Sannomiya

5. Nissan Chemical Industries, Ltd.
    Representative Director: Misao Kusano

6. Toyo Soda Manufacturing Co., Ltd.
    President: Toshiaki Yamaguchi

0195087

Format 2

NOTICE OF REFUSAL TO RECEIVE DEPOSITION

Notice No.: 60 FERM Doc. No. 271
Date of notice: March 9, 1985

Esq. Foundation Sagami Chemical Research Center
Kiyosi Kondo, Vice chairman, and other 5 (as
per attached sheet) President of Institute of Fermentation Research, Agency of
Industrial Science & Technology
Jiro Oyama (sealed)

We hereby notice that the microorganism applied by
you to be deposited cannot be received for the reason
shown below.

Remarks

1. Date of application:
   February 27, 1985

2. Representation for discrimination of microorganism:
   Escherichia coli C600/pYTP102P

3. Reason for refusal:
   Because of outside of the range of microorganisms
which can be received.

0195087

Attached sheet

Names of depositors:

1. Foundation Sagami Chemical Research Center
     Vice chairman: Kiyosi Kondo

2. Central Glass Co., Ltd.
     President: Mitsuyoshi Ito

3. Hodogaya Chemical Co., Ltd.
     President: Kenichi Fujioka

4. Nippon Soda Co., Ltd.
     President: Takeo Sannomiya

5. Nissan Chemical Industries, Ltd.
     Representative Director: Misao Kusano

6. Toyo Soda Manufacturing Co., Ltd.
     President: Toshiaki Yamaguchi

0195087

Format 2

NOTICE OF REFUSAL TO RECEIVE DEPOSITION

Notice No.: 60 FERM Doc. No. 272
Date of notice: March 9, 1985

Esq. Foundation Sagami Chemical Research Center
Kiyosi Kondo, Vice chairman, and other 5 (as
per attached sheet) President of Institute of Fermentation Research, Agency of
Industrial Science & Technology
Jiro Oyama (sealed)

We hereby notice that the microorganism applied by
you to be deposited cannot be received for the reason
shown below.

Remarks

1. Date of application:
   February 27, 1985

2. Representation for discrimination of microorganism:
   Escherichia coli C600/pYTP201N

3. Reason for refusal:
   Because of outside of the range of microorganisms
which can be received.

0195087

Attached sheet

Names of depositors:

1. Foundation Sagami Chemical Research Center
    Vice chairman: Kiyosi Kondo

2. Central Glass Co., Ltd.
    President: Mitsuyoshi Ito

3. Hodogaya Chemical Co., Ltd.
    President: Kenichi Fujioka

4. Nippon Soda Co., Ltd.
    President: Takeo Sannomiya

5. Nissan Chemical Industries, Ltd.
    Representative Director: Misao Kusano

6. Toyo Soda Manufacturing Co., Ltd.
    President: Toshiaki Yamaguchi

0195087

Format 2

## NOTICE OF REFUSAL TO RECEIVE DEPOSITION

Notice No.: 60 FERM Doc. No. 273

Date of notice: March 9, 1985

Esq. Foundation Sagami Chemical Research Center
Kiyosi Kondo, Vice chairman, and other 5 (as
per attached sheet) President of Institute of Fermentation Research, Agency of
Industrial Science & Technology
Jiro Oyama (sealed)

We hereby notice that the microorganism applied by you to be deposited cannot be received for the reason shown below.

Remarks

1. Date of application:
   February 27, 1985

2. Representation for discrimination of microorganism:
   Escherichia coli C600/pYTP301K

3. Reason for refusal:
   Because of outside of the range of microorganisms which can be received.

0195087

Names of depositors:

1. Foundation Sagami Chemical Research Center
   Vice chairman: Kiyosi Kondo

2. Central Glass Co., Ltd.
   President: Mitsuyoshi Ito

3. Hodogaya Chemical Co., Ltd.
   President: Kenichi Fujioka

4. Nippon Soda Co., Ltd.
   President: Takeo Sannomiya

5. Nissan Chemical Industries, Ltd.
   Representative Director: Misao Kusano

6. Toyo Soda Manufacturing Co., Ltd.
   President: Toshiaki Yamaguchi

Claims

1. A cyclic double-stranded DNA, comprising a high expression promoter-operator, a DNA region coding for the amino group end portion of the metapyrocatechase gene containing the SD sequence of the metapyrocatechase gene positioned in the downstream region under the control thereof and a DNA portion subsequent thereto coding for a peptite corresponding to at least the amino acid number 92 to 105 of the capsid protein VP1 of the poliovirus attenuated strain Sabin type 1, Sabin type 2 or Sabin type 3.

2. A cyclic double-stranded DNA according to Claim 1, containing a temperature sensitive gene portion which is positioned upstream on the 5'-side of the high expression promoter-operator in the opposite direction thereto and regulates its activity negatively.

3. A cyclic double-stranded DNA according to Claim 2, containing a portion coading for ampicillin resistance downstream of the temperature sensitive gene portion.

4. A cyclic double-stranded DNA according to Claim 1, wherein the high expression promoter-operator is $P_L$ promoter-operator, lac UV5 promoter-operator, trp promoter-operator or tac promoter-operator.

5. A cyclic double-stranded DNA according to Claim 2, wherein the high expression promoter-operator is $P_L$ promoter-operator.

6. A cyclic double-stranded DNA, comprising a high expression promoter-operator, a SD sequence positioned in downstream region under the control thereof, a translation initiation codon, a DNA portion connected thereto in series so as to coincide in reading frame coding for a peptide corresponding to at least the the amino acid number 92 to 105 of the capsid protein VP1 of the poliovirus attenuated strain Sabin type 1, Sabin type 2 or Sabin type 3 and a translation termination codon subsequent thereto.

7. A cyclic double-stranded DNA according to Claim 6, wherein the SD sequence is the SD sequence of the metapyrocatechase gene.

8. A cyclic double-stranded DNA according to Claim 6 or Claim 7, wherein the high expression promoter-operator is $P_L$ promoter-operator, and which is a plasmid containing a temperature sensitive gene portion for regulating its activity on the 5'-side upstream thereof.

9. A process for preparing a cyclic double-stranded DNA, comprising a high expression promoter-operator, a SD sequence positioned in downstream region under the control thereof, a translation initiation codon, a DNA portion connected thereto in series so as to coincide in reading frame coding for a peptide corresponding to at least the the amino acid number 92 to 105 of the capsid protein VP1 of the poliovirus attenuated strain Sabin type 1, Sabin type 2 or Sabin type 3 and a translation termination codon subsequent thereto, which comprises cleaving an appropriate restriction endonuclease cleavage site after said translation initiation codon of the DNA containing the high expression promoter-operator, the SD sequence positioned in downstream region under the control thereof and the translation initiation codon with said restriction endonuclease, and then, after optionally applying the treatment of smoothening the ends, inserting a DNA fragment coding for a peptide corresponding to at least the amino acid number 92 to 105 of the capsid protein VPl of the poliovirus attenuated strain Sabin type 1, Sabin type 2 or Sabin type 3 after said translation initiation codon.

10. A process according to Claim 9, wherein the SD sequence is the SD sequence of the metapyrocatechase gene.

11. A process for preparing a cyclic double-stranded DNA, comprising a high expression promoter-operator, a SD sequence positioned in downstream region under the control thereof, a translation initiation codon, a DNA portion connected thereto in series so as to coincide in reading frame coding for a peptide corresponding to at least the the amino acid number 92 to 105 of the capsid protein VPl of the poliovirus attenuated strain Sabin type 1, Sabin type 2 or Sabin type 3 and a translation termination codon subsequent thereto, which comprises imparting a translation termination codon to the carboxylic groun end by inserting an oligonucleotide designed so that the translation termination codons may be arranged at three frames at an appropriate restriction endonuclease corresponding to at least the the amino acid number 92 to 105 of the capsid protein VPl of the poliovirus attenuated strain Sabin type 1, Sabin type 2 or Sabin type 3, subjecting this to cleavage at an appropriate restriction endonuclease cleavage site after said translation initiation codon of the DNA containing the high expression promoter-operator, the SD sequence positioned in downstream region under the control thereof and the translation initiation codon with said restriction endonuclease, and then, after optionally applying the treatment of smoothening the ends, inserting the DNA obtained after said translation initiation codon.

12. A process according to Claim 11, wherein the SD sequence is the SD sequence of the metapyrocatechase gene.

13. A bacterium of the genus _Escherichia_, comprising as the plasmid a cyclic double-stranded DNA containing $P_L$ promoter-operator, a DNA region coding for the amino group end portion of the metapyrocatechase gene containing the SD sequence of the metapyrocatechase gene positioned in the downstream region under the control thereof and a DNA portion subsequent thereto coding for a peptite corresponding to at least the amino acid number 92 to 105 of the capsid protein VP1 of the poliovirus attenuated strain Sabin type 1, Sabin type 2 or Sabin type 3.

14. A bacterium of the genus _Escherichia_, which comprises retaining a cyclic double-stranded DNA containing a high expression promoter-operator, a SD sequence positioned in downstream region under the control thereof, a translation initiation codon, a DNA portion connected thereto in series so as to coincide in reading frame coding for a peptide corresponding to at least the the amino acid number 92 to 105 of the capsid protein VP1 of the poliovirus attenuated strain Sabin type 1, Sabin type 2 or Sabin type 3 and a translation termination codon subsequent thereto.

15. A bacterium according to Claim 14, wherein the SD sequence is the SD sequence of the metapyrocatechase gene.

16. A bacterium according to Claim 14 or Claim 15, retaining a cyclic double-stranded DNA wherein the high expression promoter-operator is $P_L$ promoter-operatore and a temperature sensitive gene portion for regulating its activity is contained upstream on the 5'-side thereof.

17. A process for preparing a protein having the antigenic determinant of the polyovirus capsid protein VP1, which comprises culturing a bacterium of the genus Escherichia transformed with a plasmid, comprising a high expression promoter-operator, a DNA region coding for the amino group end portion of the metapyrocatechase gene containing the SD sequence of the metapyrocatechase gene positioned in the downstream region under the control thereof, a DNA portion subsequent thereto coding for a peptite corresponding to at least the amino acid number 92 to 105 of the capsid protein VP1 of the poliovirus attenuated strain Sabin type 1, Sabin type 2 or Sabin type 3 and a portion coding for ampicillin resistance positioned upstream thereof, in a nutrient medium, and harvesting the fused protein accumulated in the microorganism cells.

18. A process according to Claim 17, wherein the high expression promoter-operator is $P_L$ promoter-operator, lac UV5 promoter-operator, trp promoter-operator or tac promoter-operator.

19. A process according to Claim 17, wherein the plasmid contains a temperature sensitive gene which controls negatiely the high expression promoter-operator between said promoter-operator and the portion coding for ampicillin resistance.

20. A process according to Claim 19, wherein the high expression promoter-operator is $P_L$ promoter-operator.

21. A process for preparing a protein having the antigenic determinant of the polyovirus capsid protein VP1, which comprises culturing a bacterium of the genus Escherichia transformed with a plasmid, comprising a high expression promoter-operator, a SD sequence positioned in downstream region under the control thereof, a translation initiation codon, a DNA portion connected thereto in series so as to coincide in reading frame coding for a peptide corresponding to at least the the amino acid number 92 to 105 of the capsid protein VP1 of the poliovirus attenuated strain Sabin type 1, Sabin type 2 or Sabin type 3 and a translation termination codon subsequent thereto, in a nutrient medium, and harvesting the VP1 protein accumulated in the microorganism cells.

22. A process according to Claim 21, wherein the SD sequence is the SD sequence of the metapyrocatechase gene.

23. A process according to Claim 21 or Claim 22, wherein the high expression promoter-operator is $P_L$ promoter-operator and a temperature sensitive gene portion for controlling its activity is contained upstream on the 5'-side thereof.

Fig. 1

Fig. 2

319

0195087

Fig. 3

Fig. 3

419

Fig. 4

0195087

Fig. 5

Fig. 6

0195087

Fig. 7

Fig. 8

0195087

Fig. 9

# INTERNATIONAL SEARCH REPORT

International Application No. PCT/JP85/00518

## I. CLASSIFICATION OF SUBJECT MATTER (if several classification symbols apply, indicate all) ³

According to International Patent Classification (IPC) or to both National Classification and IPC

Int.Cl⁴ C12N15/00, C12N1/20, C12P21/00//
A61K39/13(C12N1/20, C12R1:19)(C12P21/00, C12R1:19)

## II. FIELDS SEARCHED

Minimum Documentation Searched ⁴

| Classification System | Classification Symbols |
|---|---|
| IPC | C12N 15/00, C12N 1/20, C12P 21.00, A61K 39/13 |

Documentation Searched other than Minimum Documentation
to the Extent that such Documents are Included in the Fields Searched ⁵

## III. DOCUMENTS CONSIDERED TO BE RELEVANT ¹⁴

| Category* | Citation of Document, ¹⁶ with indication, where appropriate, of the relevant passages ¹⁷ | Relevant to Claim No. ¹⁸ |
|---|---|---|
| A | JP, A, 59-501866 (The National Biological Standards) 8 November 1984 (08. 11. 84), & WO, A, 84/01575 & GB, A, 2128621 & EP, A1, 107436 & EP, A1, 120906 | 1 - 23 |
| A | JP, A, 59-173084 (Institut Pasteur), 29 September 1984 (29. 09. 84) & EP, A2, 110791 | 1 - 23 |
| A | JP, A, 58-500759 (Institut Pasteur) 12 May 1983 (12. 05. 83) & WO, A, 82/04067 & EP, A1, 65924 & EP, A1, 79353 & FR, B, 2506326 | 1 - 23 |
| A | JP, A, 60-207582 (Zaidan Hojin Nippon Porio Kenkyusho) 19 October 1985 (19. 10. 85) (Family: none) | 1 - 23 |
| A | A, Nomoto et al., Proc. Natl. Acad. Sci., USA, Vol. 79, 1982, pages 5793-5797 | 1 - 23 |

* Special categories of cited documents: ¹⁵
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance: the claimed invention cannot be considered novel or cannot be considered to involve an inventive step
"Y" document of particular relevance: the claimed invention cannot be considered to involve an inventive step when the document is combined with one or more other such documents, such combination being obvious to a person skilled in the art
"&" document member of the same patent family

## IV. CERTIFICATION

| Date of the Actual Completion of the International Search ² | Date of Mailing of this International Search Report ² |
|---|---|
| December 12, 1985 (12.12.85) | December 23, 1985 (23.12.85) |

| International Searching Authority ¹ | Signature of Authorized Officer ²⁰ |
|---|---|
| Japanese Patent Office | |

Form PCT/ISA/210 (second sheet) (October 1981)